Europäisches Patentamt

European Patent Office

Office européen des brevets

(11) Veröffentlichungsnummer: **0 026 532**
**B1**

(12) **EUROPÄISCHE PATENTSCHRIFT**

(45) Veröffentlichungstag der Patentschrift:
04.06.86

(21) Anmeldenummer: **80200887.0**

(22) Anmeldetag: **22.09.80**

(51) Int. Cl.⁴: **A 61 K 33/00,** A 61 K 7/48,
A 61 K 7/00, A 61 K 33/30,
A 61 K 33/34, A 61 K 33/38,
A 61 K 33/24 // (A61K33/38,
33:34, 33:00, 33:30, 33:24, 31:18)

(54) **Haut- und Schleimhautpräparat, Verfahren zu seiner Herstellung und Mittel zur Durchführung des Verfahrens.**

(30) Priorität: **27.09.79 CH 8713/79**

(43) Veröffentlichungstag der Anmeldung:
**08.04.81 Patentblatt 81/14**

(45) Bekanntmachung des Hinweises auf die Patenterteilung:
**04.06.86 Patentblatt 86/23**

(84) Benannte Vertragsstaaten:
**AT BE CH DE FR GB IT LI NL SE**

(56) Entgegenhaltungen:
US - A - 3 920 835
US - A - 3 988 470

FORMULAIRE PHARMACEUTIQUE, Vigot Frères, 1965,
Jean Leclerc, Paris, FR. "Acide nucléinique", Seite 40

(73) Patentinhaber: **Solco Basel AG, Geilertstrasse 18,
CH-4052 Basel (CH)**

(72) Erfinder: **Mardi, Shalva, Bleicherweg 3,
CH-4102 Binningen (CH)**
Erfinder: **Lichti, Heinz Felix, Spechtweg 9,
CH-4125 Riehen (CH)**
Erfinder: **Baumgartner, Guido, Hauptstrasse 28,
CH-4126 Bettingen (CH)**
Erfinder: **Garteiz, Daniel, 8787 Sturbridge Drive,
Cincinnati OH 45236 (US)**
Erfinder: **Judd, Claude Ivan, 6149 Kilrenny Drive,
Loveland OH 45140 (US)**
Erfinder: **Weiner, Murray, 8915 Spooky Ridge Lane,
Cincinnati OH 45242 (US)**

(74) Vertreter: **Frossard, Michel, Dr. et al, A. Braun, Braun,
Héritier, Eschmann AG, Patentanwälte
Holbeinstrasse 36-38, CH-4051 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Seit Jahrhunderten schon sind ätzende Präparate, insbesondere starke Säuren, zum «Ausbrennen» von Hautfehlern, Warzen und dergleichen verwendet worden. Hierbei sind jedoch nicht alle starken Säuren gleich beliebt – dies vermutlich wegen der recht unterschiedlichen Natur ihrer Wirkungen auf die integumentalen Proteine. So haben sich insbesondere konzentrierte Formen der Salpetersäure, ferner die Salicylsäure und gewisse Halogenessigsäuren [H.W. Felter, The Eclectic Materia Medica Pharmacology and Therapeutics, John K. Scudder Publisher, Cincinnati (OH, USA) 1922, Seite 133; A.L. Welsch, The Dermatologist's Handbook, C.C. Thomas Publisher, Springfiled (USA) 1957, Seite 111; C.J. Lunsford u. Mitarb., Arch. Dermat. and Syph. 68 (1953), 148] in dem Arzneimittelschatz der Dermatologen einen mehr oder weniger spezifischen Platz verschafft, während hingegen Salzsäure selten verwendet wird.

Ebenfalls zur Beseitigung von Warzen wurden auch schon die Milchsäure und die Oxalsäure (H.W. Felter, loc. cit.) sowie auch Essigsäure vorgeschlagen (F.P. Foster, Practical Therapeutics, D. Appleton and Co., 1897, Seite 226). Die keratolytische Wirkung dieser Säuren ist jedoch gering, und sie werden daher fast immer in Kombination mit Salicylsäure benutzt (Deutsche Auslegeschrift 1 266 448). Beispielsweise wird in den USA unter dem Namen Compound W Wart Remover ein Präparat verkauft, welches Essigsäure und Salicylsäure enthält (Handbook of Nonprescription Drugs, 5. Auflage, American Pharmaceutical Association 1977, Seiten 364 und 368). In neuerer Zeit wurde in den US-Patentschriften 3 920 835 und 3 988 470 ein Präparat aus Glykolsäure, Citronensäure, Äpfelsäure, Tartronsäure, Weinsäure, Glucuronsäure, Brenztraubensäure, Methylpyruvat, Äthylpyruvat, 2-Hydroxiisobuttersäure oder 3-Hydroxibuttersäure beschrieben, mit welchem auf übersteigerter Keratinbildung beruhende Hautkrankheiten, z.B. die Akne und die palmare oder plantare Hyperkeratose, behandelt werden können.

Bekanntlich hinterlassen aber die Behandlungen mit ätzenden Präparaten öfters mehr oder weniger hässliche Narben (R. Volk und F. Winter, Lexikon der kosmetischen Praxis, Verlag von Julius Springer, Wien 1936, Seite 677; K.O. Møller, Pharmakologie, 2. deutsche Auflage, Benno Schwabe & Co, Basel 1953, Seiten 167 und 584).

Die therapeutische Anwendung ätzender Präparate zur Behandlung des Hautkrebses wird ausdrücklich abgelehnt (R. Volk und F. Winter, loc. cit., Seite 308), weil die Gefahr einer nicht vollständigen Beseitigung der Karzinome zu gross ist; danach sieht man recht oft, dass ein ungenügend behandeltes Karzinom plötzlich rasch zu wuchern beginnt.

Andererseits sind im 19. Jahrhundert zur Beseitigung von Warzen und anderen Hautfehlern verschiedene Metallsalze vorgeschlagen worden, darunter Kupfersalze wie das Acetat und das Sulfat, Bleisalze in Kombination mit Zinksulfat, Kupfersulfat und Essig, ferner Antimon-, Arsen-, Chrom-, Quecksilber- und Silbersalze (C.J. Lunsford, loc. cit.) und Cadmiumsalze [S.O.L. Potter, Therapeutic Materia Medica and Pharmacy, P. Blakiston's Son and Co., Philadelphia (Pa, USA) 1909, Seite 185]. Von allen diesen Salzen hat lediglich Zinkchlorid in Verbindung mit Trichloressigsäure – Methode von Mohs – zur Behandlung vom Hautkrebs eine vorübergehende Bedeutung erlangt.

Schliesslich wird im Erfinderschein 229 744 der UdSSR (erteilt 1969; A.Z. Karchauli) ein Mittel zur Behandlung benigner Tumoren und präkanzeröser Zustände der Haut beschrieben, welches aus einer Mischung von Kupfernitrat und Milchsäure besteht. Die Herstellung erfolgt durch Vermischen von Kupfernitrat enthaltender Salpetersäure und Milchsäure im Verhältnis 1 : 2 und 3 : 1. Die experimentelle Nacharbeitung dieses Mittels allerdings hat eine Unbeständigkeit erwiesen, die bis zur Explosion des Gefässes hin führen kann.

Es wurde nun ein neues Präparat gefunden, mit welchem oberflächliche Haut- und Schleimhautveränderungen benigner und prämaligner Art und Basaliome erfolgreich behandelt werden können; das Präparat entfaltet seine Wirkung bei topischer Anwendung.

Unter die erwähnten Veränderungen fallen zunächst Veränderungen und Wucherungen der Haut und der Schleimhäute, welche der körperlichen Schönheit abträglich sind, im Volksmund jedoch keine Krankheiten darstellen, z.B. Warzen, Muttermale, Pigmentmale, Blutschwamm und Altersschwielen, dann aber gutartige Hautkrankheiten und schliesslich präkanzeröse Zustände und Basaliome.

Erfindungsgemäss besteht das Präparat aus einer wässrigen Lösung, welche (1) Salpetersäure in einer Konzentration und Menge, welche der Lösung einen pH-Wert unterhalb von etwa 1 und ein Säureäquivalent von etwa 6 bis 10 Millimol/ml verleihen, und (2) ein in wässriger Salpetersäure lösliches Metallnitrit oder salpetrige Säure in einer Menge, welche etwa 0,01 bis 5 mg Nitrit ($NO_2^-$) per ml entspricht, enthält. Dieses Präparat ist bei Aufbewahrung bei Raum- oder tieferer Temperatur inbezug auf die angegebenen Konzentrationsbereiche der Komponenten 1 und 2 beständig.

Da es sich bei dem neuen Präparat um eine stark saure Lösung handelt, waren Vergleiche mit anderen Säuren oder stark sauren Präparaten angebracht, um daraus die jeweilige Rolle seiner wesentlichen Merkmale und die Gründe seiner hervorragenden Wirkung besser zu verstehen. Zu diesem Zweck sind zwei spezifische biologische Teste eigens entwickelt worden. Wie in der Folge ausführlich gezeigt werden soll, gehen in der Tat die kosmetische und therapeutische Wirksamkeit mit positiven Ergebnissen in diesen Testen streng parallel.

## I. In vitro-Test

Wird weisses menschliches oder tierisches Haar, z.B. Rattenhaar, in das Präparat eingetaucht, so verfärbt es sich rasch in ein leuchtendes Gelb, während die Lösung selbst farblos bleibt; das Haar behält dabei seine strukturelle Unversehrtheit. Diese «Fixierung» des Haares mit gleichzeitiger Farbänderung spiegelt offenbar eine chemische Reaktion wieder, zufolge welcher das Gewebe nicht mehr lebensfähig ist, aber seine anatomische Struktur weitgehend beibehält; das Gewebe ist also mumifiziert.

Im Gegensatz dazu bewirkt Salzsäure in einer Konzentration von 20 bis 35% (Gewicht/Gewicht) keine Verfärbung des Haares, wohl aber seine Auflösung innert weniger Stunden (je nach angewendeter Konzentration). Auch 10%ige Essigsäure – ein zur lokalen Behandlung der Warzen anerkanntes Mittel – bewirkt keine gelbe Verfärbung des Haares.

## II. In vivo-Test

Wie jeder Chemiker aus eigener Erfahrung weiss, bewirkt der Kontakt der Haut mit konzentrierter Salpetersäure eine gelb-braune Verfärbung, welche erst nach einer Woche oder mehr verschwindet. Mit 33,4%iger Salpetersäure erfolgt bei einer Einwirkungsdauer von weniger als 1 Minute keine oder eine kaum sichtbare Reaktion der Haut. Wird die Säure 2 Minuten einwirken lassen, so wird ein stechendes, einige Minuten währendes Gefühl, aber keine sofortige andere Reaktion wahrgenommen; nach mehreren Minuten entsteht ein Erythem, das nach 15 bis 45 Minuten sein Maximum erreicht und dann nach einer bis zwei Stunden spurlos verschwindet.

Wird nun das Präparat auf normale menschliche Haut aufgetragen und während kurzer Zeit einwirken lassen, so erscheint sofort danach eine charakteristische braune Verfärbung (englisch: tan), die während Tagen bestehen bleibt. Mitunter tritt in einer schmalen Randzone ein leichtes, vorübergehendes Erythem auf. Zweckmässig werden 10 µl (0,01 ml) der Lösung auf die Unterseite des Vorderarmes aufgetragen und nach bestimmter Einwirkungsdauer, in der Regel 1 bis 3 Minuten, weggetupft.

Vergleicht man in diesen zwei Testen das Präparat und Salpetersäurelösungen derselben und anderer Konzentrationen, so ergibt sich daraus folgendes Bild:

Tabelle 1
(Rattenhaar)

| | |
|---|---|
| Salpetersäure 33,4%* (6,2 normal), allein | gelbe Verfärbung nach mindestens 30 Minuten |
| idem, + 3   mg/ml NaNO$_2$ [erfindungsgemäss] | gelbe Verfärbung in 1 bis 2 Minuten |
| idem, + 8,9 mg/ml Cd(NO$_2$)$_2$ [erfindungsgemäss] | gelbe Verfärbung, 1-2 Minuten** |
| idem, + 0,6 mg/ml NaNO$_2$ [erfindungsgemäss] | gelbe Verfärbung, 2-3 Minuten |
| | |
| Salpetersäure 17,8% (3,1 normal), allein | keine Farbänderung in 48 Stunden |
| idem, + 3 mg/ml NaNO$_2$ | gelbe Verfärbung, 10-13 Minuten |
| | |
| Salpetersäure 6,1% (1,0 normal), allein | keine Farbänderung in 48 Stunden |
| idem, + 3 mg/ml NaNO$_2$ | sehr schwache gelbe Verfärbung, 116-134 Min., keine Steigerung der Farbintensität in 48 Std. |
| | |
| konz. HNO$_3$ (65%; 14,3 normal), allein | gelbe Verfärbung in 1-2 Minuten, rasche Auflösung des Haares unter Bildung einer klaren gelben Lösung |
| | |
| NaNO$_2$, 10 mg/ml, allein | keine Farbänderung in 48 Stunden |

\* Sämtliche %-Angaben beziehen sich auf Gewicht/Gewicht
\*\* Die «Zeitspannen» in Minuten bedeuten jeweils Zeitpunkt der letzten negativen Beobachtung – Zeitpunkt der ersten positiven Beobachtung

Tabelle 2
(menschliche Haut, Unterseite des Vorderarmes)

| A. 3 Minuten Einwirkungsdauer | | |
|---|---|---|
| | nach Minuten: | nach Tagen: |
| Salpetersäure 33,4% | rosa Verfärbung (Erythem) | keine Spuren mehr |
| idem, + 3 mg/ml NaNO$_2$ [erfindungsgemäss] | braune Verfärbung | hellbraune Verfärbung |

B. 2 Minuten Einwirkungsdauer

| | sofort danach: | nach 1 Stunde: | nach 2¾ Stunden: | nach 7 Stunden: |
| --- | --- | --- | --- | --- |
| Salpetersäure 33,4% | keine Reaktion | deutlicher rosa Fleck (Erythem) | rosa Fleck, knapp sichtbar | keine Spuren mehr |
| idem, + 8,9 mg/ml Cd(NO₂)₂ [erfindungsgemäss] | brauner Fleck | | der braune Fleck bleibt bestehen* | |

$*$ Auch nach 48 Stunden praktisch unverändert

Aus den Tabellen gehen die eigenartige, bisher völlig unbekannte biologische Wirkung des erfindungsgemässen Präparates und die auffallende Rolle der salpetrigen Säure eindeutig hervor. Gegenüber einer vergleichbaren Lösung reiner Salpetersäure lassen die Teste einen signifikanten Unterschied zum Vorschein kommen, der sich in den Ergebnissen der klinischen Prüfung wiederspiegelt.

Insgesamt bewirkt das Präparat eine – gemessen an der Verfärbung des Haares und der Haut – gleich rasche chemische Reaktion wie konzentrierte Salpetersäure, jedoch ohne die kräftige ätzende, alle Gewebe undifferenziert auflösende Wirkung dieser und anderer starker Säuren. Durch die Wirkung des Präparates werden sofort die integumentalen Proteine in situ denaturiert und die anatomische Struktur wird ohne Verletzung intravital fixiert (Mumifizierung).

Die bemerkenswerte Funktion des Metallnitrites bzw. der salpetrigen Säure im Präparat lässt sich auch eindrücklich dadurch belegen, dass man der Lösung Harnstoff zusetzt, welcher bekanntlich die salpetrige Säure zersetzt. Bei mengenmässig ausreichendem Harnstoffzusatz wird die positive Reaktion bei dem in vitro-Test ganz unterdrückt.

Der negative Einfluss des Harnstoffes lässt sich im in vivoTest ebenfalls feststellen. Werden nämlich dem in Tabelle 3 erwähnten, erfindungsgemässen Präparat 10 mg Harnstoff per ml der Lösung vor dem Auftragen auf menschliche Haut zugegeben, so tritt keine Farbreaktion ein.

Tabelle 3
(Rattenhaar)

| Salpetersäure 33,4% + 3 mg/ml NaNO₂ [erfindungsgemäss] | gelbe Verfärbung in 1-2 Minuten |
| --- | --- |
| gleiches Präparat + 2 mg/ml Harnstoff | gelbe Verfärbung, 4-5 Minuten |
| gleiches Präparat + 10 mg/ml Harnstoff | gelbe Verfärbung, 234-264 Minuten |

Gemäss einer vorteilhaften Ausführungsform der Erfindung weist die wässrige Lösung ein Säureäquivalent von etwa 7,0 bis etwa 8,5 Millimol/ml auf; ausserdem liegt ihr pH-Wert vorzugsweise unterhalb von etwa 0. Die Lösung enthält in der Regel etwa 300 bis etws 600 mg reine Salpetersäure per ml.

Als Metallnitrit eignen sich u.a. Natrium- und Kaliumnitrit wegen ihrer leichten Verfügbarkeit und Dosierbarkeit. Während die untere Grenze an Komponente (2), als Nitrit ($NO_2^-$) berechnet, um 0,01 mg/ml liegt, lässt sich eine obere Grenze nicht eindeutig festlegen. Wie Tabelle 4 zeigt, erscheint eine Erhöhung des Nitritgehaltes über 3 mg/ml nicht sinnvoll, weil dadurch keine raschere Verfärbung des Rattenhaares erreicht wird. Erfindungsgemäss darf eine Höchstmenge von 5 mg/ml nicht überschritten werden.

Tabelle 4
(Rattenhaar)

| Zugabe von Natriumnitrit zu Salpetersäure 33,4% | | | | | | | |
| --- | --- | --- | --- | --- | --- | --- | --- |
| NaNO₂ in mg/ml | 0 | 0,08 | 0,5 | 1,5 | 3,0 | 4,0 | 5,0 |
| gelbe Verfärbung (in Minuten) | 202-242 | 146-206 | 15-22 | 2-3 | 1-2 | 1-2 | 1-2 |

Aus der Tabelle ergibt sich die minimale Wirksamkeit des Präparates bei einem Nitritgehalt im Bereiche von 0,1 bis 0,5 mg/ml, gemessen an der Reaktion eines gesunden und festen Gewebes (Rattenhaar). Wie später noch gezeigt wird, erfolgt aber die Reaktion einer Hautläsion oder eines pathologisch veränderten Gewebes rascher und stärker als jene eines normalen Integuments. Deshalb dürfte der minimalen Wirksamkeit bei in vitro-Test eine bei der klinischen und kosmetischen Anwendung optimale Wirksamkeit entsprechen. Bevorzugt wird daher ein Präparat mit einem Nitritgehalt von etwa 0,1 bis 0,5 mg/ml.

Überraschenderweise hat es sich als besonders vorteilhaft, im Hinblick auf die Zusammensetzung und damit auch auf die Wirkung, erwiesen, wenn das Präparat ausserdem eine durch Salpetersäure oxidierbare organische Carbonsäure enthält. Als solche eignen sich z.B. Oxalsäure und aliphatische Hydroxisäuren, Ketosäuren und ungesättigte Carbonsäuren, wie z.B. Milchsäure, Glykolsäure, Glyoxylsäure, Äpfelsäure, Weinsäure, Brenztraubensäure, Maleinsäure, Fumarsäure, Dimethylmaleinsäure, 2-Hydroxybuttersäure, Tartronsäure, Mesoxalsäure, Citronensäure, Citraconsäure und Glucuronsäure.

Mit der Salpetersäure bilden solche organische Säuren Reduktionsprodukte, wie nitrose Gase und salpetrige Säure, in ausreichender Menge, um im Präparat die erforderliche Mindestkonzentration an Nitrit zu gewährleisten und damit auch die erfindungsgemässe chemische Zusammensetzung unter allen Umständen aufrechtzuerhalten. Bekanntlich ist die Oxidationsgeschwindigkeit oxidierbarer organischer Säuren unterschiedlich gross, ihrer chemischen Struktur entsprechend; beispielsweise wird Brenztraubensäure durch Salpetersäure rasch, Oxalsäure hingegen langsam oxidiert, während Milchsäure diesbezüglich eine Mittelstellung einnimmt. Es ist also vorteilhaft, dem Präparat ein Gemisch von unterschiedlich rasch oxidierbaren organischen Säuren, zweckmässig Brenztraubensäure, Milchsäure und Oxalsäure, zuzusetzen; dadurch wird über eine längere Zeitspanne der gewünschte Nitritgehalt sichergestellt.

Die eigenartige Rolle der oxidierbaren Carbonsäuren für die Aufrechterhaltung der Zusammensetzung innerhalb der angegebenen Grenzwerte erschöpft sich jedoch nicht in der Neubildung von salpetriger Säure durch die erwähnte Redoxreaktion. Aus diesen, der Salpeter- und der salpetrigen Säure bilden sich nämlich, vor allem bei dem weiter unten besprochenen oxidativen Herstellungsverfahren, gewisse Kondensationsprodukte, so etwa Ester und Anhydride – z.B. O-Nitryl- und O-Nitrosylserivate der Milchsäure und der O-Lactylmilchsäure. Diese Verbindungen erweisen sich als relativ unbeständig, d.h. sie neigen zu einem langsamen Zerfall, unter Rückbildung der salpetrigen Säure, nach Massgabe des Gleichgewichtszustandes in der Lösung und üben dadurch eine stabilisierende Wirkung aus.

Überhaupt kann die wässrige Lösung, nebst den oxidierbaren Carbonsäuren, weitere organische Säuren enthalten, welche eine keratolytische Wirkung entfalten. Hierzu eignen sich u.a. Essigsäure, die Halogenessigsäuren, Salicylsäure usw. Eine eigene keratolytische Wirkung des Präparates kommt allerdings meistens schon dadurch zustande, dass gewisse oxidierbare Carbonsäuren, z.B. Brenztraubensäure und Milchsäure, an sich diese Wirkung besitzen.

Vorzugsweise enthält also das Präparat Milchsäure, z.B. in einer Menge von ca. 4 bis 40 mg/ml, Oxalsäure, z.B. in einer Menge von 20 bis 60 mg/ml, und Essigsäure, z.B. in einer Menge von ca. 10 bis 50 mg/ml, gegebenenfalls auch Brenztraubensäure, z.B. in einer Menge von ca. 1 mg/ml.

Als andere fakultative Komponente kann die wässrige Lösung mit Vorteil ein Metallsalz enthalten, welches bei äusserlicher Anwendung nicht toxisch und in salpetersaurer Lösung bei einem pH unterhalb von ca. 1 löslich ist. Unter solchen Metallsalzen kommen in Frage insbesondere jene von Kupfer, Silber, Cadmium, Zink, Aluminium, Calcium, Strontium, Magnesium, Eisen – vorzugsweise Eisen(III), Antimon, Wismuth, Selen, Mangan, Zirkonium, Cobalt, Gold, Titan und Zinn. Bevorzugt werden die entsprechenden Nitrate.

Von den genannten Metallionen haben sich die Kupfer-, Silber-, Cadmium- und Zinkionen als besonders geeignet erwiesen; bei den Kupferionen wird $Cu^{++}$ aus praktischen Gründen bevorzugt. Dabei kann die wässrige Lösung bis zu ca. 0,1 mg Kupferion per ml, oder bis zu ca. 6 mg Silberion per ml, oder bis zu ca. 3,5 mg Cadmiumion per ml, oder bis zu ca. 6 mg Zinkion per ml enthalten. Es können auch Zusätze von mehr als einem dieser Metallionen gleichzeitig vorliegen, beispielsweise von Kupfer- und Cadmiumionen, von Silber- und Zinkionen, von Cadmium- und Zinkionen, von Kupfer- und Silberionen oder von Kupfer-, Cadmium- und Zinkionen.

Bevorzugt wird ganz besonders jene Zusammensetzung des Präparates, bei der die wässrige Lösung – nebst der Salepetersäure und der salpetrigen Säure – Essigsäure, Oxalsäure, Milchsäure sowie Cadmiumionen und Kupferionen enthält, wobei diese Komponenten vorzugsweise in den zuvor angegebenen Konzentrationen vorliegen und die Lösung den bereits erwähnten pH-Wert und das angegebene Säureäquivalent aufweist. Ein solches Präparat wird in den Beispielen 2 und 7 bis 10 genauer beschrieben.

Die Herstellung des Präparates kann einfach durch das Mischverfahren erfolgen, d.h. durch blosse Vermischung der Komponenten miteinander. Bei der oben beschriebenen, bevorzugten Zusammensetzung kann die Auflösung wegen der unterschiedlichen Löslichkeiten der Ausgangsprodukte längere Zeit benötigen; zwecks Beschleunigung des Vorgangs ist es daher von Vorteil, während der Zugabe ständig zu rühren. Zweckmässig werden zuerst die organischen Säuren und die anorganischen Salze, vorzugsweise Nitrate, in Wasser aufgelöst und die wässrige Lösung dann langsam mit 65%iger Salpetersäure (spezifisches Gewicht ca. 1,41) versetzt; hierauf wird die Lösung durch Verdünnung mit Wasser auf die gewünschte Konzentration gebracht und eventuell mit Natriumnitrit versetzt. Wenn sich aus der Lösung nach einigen Tagen Kristalle ausgeschieden haben, werden sie durch Filtration entfernt.

Bevorzugt wird allerdings die Herstellung durch ein oxidatives Verfahren, bei dem das Nitrit nicht zugegeben sondern in der Lösung durch Oxidation der organischen Carbonsäuren in reichlicher Menge gebildet wird. Zudem entstehen dabei aus letzteren und den nitrosen Gasen bzw. salpetriger Säure Kondensationsprodukte in genügender Menge, um die vorher besprochene stabilisierende oder selbstregenerierende Wirkung zu gewährleisten. Man hat zwei Präparate vergleichbarer Zusammensetzung durch das Mischverfahren bzw. das oxidative Verfahren hergestellt und über eine Zeitspanne von 13 bis 111 Tagen (bei Raumtemperatur) mit dem biologischen in vitro-Test (Rattenhaar) regelmässig geprüft; die Geschwindigkeit der gelben Verfärbung zeigte bei dem «oxidativen» Produkt kaum eine Abnahme, bei dem Produkt aus dem Mischverfahren hingegen war der Aktivitätsverlust deutlich grösser.

Die Selbstregenerierung, insbesondere des oxidativ hergestellten Präparates, lässt sich mit dem in vitro-Test leicht verfolgen, wie in folgender Tabelle am Präparat von Beispiel 7 gezeigt wird.

Tabelle 5
(Rattenhaar)

| Bedingungen der Aufbewahrung | Zeitpunkt der Prüfung | Gelbe Verfärbung des Rattenhaares, in Min. |
|---|---|---|
| verschlossenes Gefäss* | zu Beginn | $\langle 1$ |
| | 13 Tage danach | $\langle 1$ |
| | 28 Tage danach | $\langle 1$ |
| Verschluss entfernt | zu Beginn | $\langle 1$ |
| | 23 Minuten danach | 1-2 |
| | 2 Stunden danach | 12-18 |
| | 5½ Stunden danach | 77-104 |
| Gefäss wieder verschlossen | 25 Stunden danach | 26-30 |

* nur zur raschen Probeentnahme geöffnet

Im Übrigen wurde auch gefunden, dass die Zusammensetzung der Präparate, namentlich inbezug auf den Nitritgehalt und den Oxalatgehalt, im Laufe der Zeit nach ähnlichen oder vergleichbaren Werten hin tendiert, ob die Herstellung durch Vermischung unter Zugabe eines Nitrites oder durch das oxidative Verfahren erfolgt ist. Wichtig dazu ist, dass die Gefässe während der Lagerung nicht hermetisch verschlossen, sondern mit einem lockeren Verschluss versehen sind. Diese langsame Konvergenz der Zusammensetzung von Präparaten verschiedener Herstellungsweise wird in Beispiel 11 ausführlich dargelegt; sie verleiht dem erfindungsgemässen Präparat eine weitgehende Einheitlichkeit innerhalb der angegebenen Grenzwerte.

Bei der Herstellung durch das oxidative Verfahren können noch andere, durch Oxidation in die gewünschten Endprodukte überführbare Ausgangsprodukte herangezogen werden, so u.a. Metalle, einfache Ester der genannten Carbonsäuren und Glucose. Wird das bevorzugte Präparat nach diesem Verfahren hergestellt, kann man grundsätzlich folgendermassen vorgehen.

Aus Salpetersäure, Kupfernitrat und Cadmiumnitrat, Essigsäure, Milchsäure und Oxalsäure wird eine Mischung hergestellt. Kupfernitrat bzw. Cadmiumnitrat können in situ durch Auflösen von metallischem Kupfer bzw. Cadmium in Salpetersäure oder in einem salpetersäurehaltigen Gemisch erzeugt werden. Essigsäure, Milchsäure und Oxalsäure können auch in Form ihrer niederen Alkylester, z.B. der Äthylester, zugegeben werden. Die Äthylester von Essigsäure, Milchsäure oder Oxalsäure können ferner, wenigstens in geringem Ausmass, durch Zugabe von Äthanol zum Gemisch der freien Säuren in situ erzeugt werden.

Allgemein lässt sich das oxidative Verfahren so durchführen, dass man eine Kupferionen und Cadmiumionen enthaltende Lösung in konzentrierter Salpetersäure in beliebiger Reihenfolge mit Milchsäure oder Milchsäureäthylester, Oxalsäure oder Oxalsäurediäthylester, Essigsäure oder Essigsäureäthylester und gegebenenfalls Äthanol versetzt und bei leicht erhöhter bis mässig erhöhter Temperatur ausreagieren lässt.

Eine bevorzugte Ausführungsform des Verfahrens besteht darin, dass man konzentrierte Salpetersäure, ein wasserlösliches Kupfer(II)-salz, vorzugsweise Kupfer(II)-nitrat, oder metallisches Kupfer und ein wasserlösliches Cadmiumsalz, vorzugsweise Cadmiumnitrat, oder metallisches Cadmium miteinander auflöst bzw. reagieren lässt, die entstandene Lösung mit Milchsäure oder Milchsäureäthylester und eventuell mit wässrigem Äthanol versetzt und reagieren lässt, die gebildete Lösung mit Oxalsäure oder Oxalsäurediäthylester und hierauf mit Essigsäure oder Essigsäureäthylester versetzt und die Lösung ausreagieren lässt. Während der ganzen Herstellungsdauer wird die Temperatur, durch Abkühlen oder Erwärmen, auf Raumtemperatur bis leicht erhöhter Temperatur, vorzugsweise 20 bis 40 °C, gehalten.

Eine weitere Ausführungsform besteht darin, dass man aus Milchsäure oder Milchsäureäthylester, Oxalsäure oder Oxalsäurediäthylester, Essigsäure oder Essigsäureäthylester, Äthanol, einem wasserlöslichen Cadmiumsalz, vorzugsweise Cadmiumnitrat, und einem wasserlöslichen Kupfer(II)-salz, vorzugsweise Kupfer(II)-nitrat, ein Gemisch herstellt; nach weitgehender Auflösung, gegebenenfalls durch Erhitzen auf mässige Temperatur, versetzt man mit konzentrierter Salpetersäure bei Raumtemperatur und lässt das Gemisch bei mässiger Temperatur ausreagieren.

Eine weitere Ausführungsform besteht darin, dass man aus Milchsäure oder Milchsäureäthylester und wasserfreiem Äthanol ein Gemisch herstellt und nacheinander mit konzentrierter Salpetersäure, Oxalsäure oder Oxalsäurediäthylester, Essigsäure oder Essigsäureäthylester, einem wasserlöslichen Cadmiumsalz, vorzugsweise Cadmiumnitrat, einem wasserlöslichen Kupfer(II)-salz, vorzugsweise Kupfer(II)-nitrat, und Wasser versetzt; in der entstandenen Lösung wird die Reaktion durch abwechslungsweises Erhitzen auf mässige Temperatur und Stehenlassen bei Raumtemperatur während längerer Zeit eingeleitet und ablaufen lassen.

Bei diesen Arbeitsweisen können die Wärmezufuhr und die Reaktionsdauer derart gewählt oder gesteigert werden, dass die Endkonzentration an Milchsäure erheblich herabgesetzt und die Haltbarkeit des Präparates bei Raumtemperatur erhöht werden. Gewünschtenfalls wird Einheitlichkeit der Endzusammensetzung bzw. deren Übereinstimmung mit den erfindungsgemässen Erfordernissen durch weitere Zugabe anderer Komponenten, wie z.B. von Oxalsäure und salpetriger Säure, erreicht.

In jedem Fall lässt man solange ausreagieren, bis die Flüssigkeit klar und farblos geworden ist und einen pH-Wert unterhalb von ca. 1 aufweist und sich praktisch keine Gasblasen mehr bilden, was im allgemeinen etwa 2 bis 3 Monate benötigt; hierauf trennt man die überstehende Flüssigkeit von dem entstandenen Bodenkörper, der verworfen wird, ab.

Beispielsweise kann man auf 900 bis 1000 ml 65%ige Salpetersäure 20 ml Eisessig, 1 g Oxalsäure-dihydrat, 205 g 90%ige Milchsäure, 20 bis 25 ml wasserfreies Äthanol, 1 g Maleinsäure und 15 ml Brenztraubensäure einsetzen.

Die quantitative Analyse der durch das oxidative Verfahren hergestellten Beispiele und einer Reihe weiterer Beispiele nach den erwähnten Ausführungsformen hat für die einzelnen Werte und Gehalte den unten stehenden Bereich ergeben; es lassen sich daraus für die Zusammensetzung und Kenndaten der Endlösung die folgenden Soll-Bereiche ableiten:

### Tabelle 6

| | Experimentell gefundener Bereich | Soll-Bereich | |
|---|---|---|---|
| Spezifisches Gewicht | 1219-1312 | 1200-13200 | mg/ml |
| Säureäquivalent | 7,38-10,3 | 6,0-10,0 | mmol/ml |
| Trockengewicht* | 632-931 | 600-950 | mg/ml |
| Gehalt an $Cd^{2+}$ | 1,34-2,15 | 1,3-2,3 | mg/ml |
| Gehalt an $Cu^{2+}$ | 0,007-0,055 | 0,006-0,060 | mg/ml |
| Oxalatgehalt | 28-45 | 25-60 | mg/ml |
| Lactatgehalt | 0-45 | 0-50 | mg/ml |
| Nitratgehalt | 349-519 | 300-600 | mg/ml |
| Nitritgehalt | 0,0001-25,93 | 0,01-5 | mg/ml |
| Essigsäuregehalt | 21-47 | 20-50 | mg/ml |
| Äthanol | 0 | 0 oder Spur | mg/ml |

* Nach Neutralisation und Lyophilisation

Das bevorzugte Präparat besteht also aus einer wässrigen Lösung, deren Komponenten obigem Soll-Bereich entsprechen; in dieser Zusammensetzung hat es sich in der kosmetischen und der klinischen Prüfung hervorragend bewährt.

Ein wolches Präparat ist ebenfalls der Prüfung in dem in vitro- und in vivo-Test unterzogen worden, wie in den folgenden Tabellen gezeigt wird. Bei der in vitro-Prüfung wird die signifikante Rolle der salpetrigen Säure sowohl durch den Vergleich mit einer entsprechenden Lösung reiner Salpetersäure als auch durch den Zusatz von Harnstoff bestätigt. Ebenso eindrücklich fällt der ähnliche Vergleich bei der in vivo-Prüfung, durch Auftragen von 0,01 ml der Lösungen auf menschliche Haut, aus.

### Tabelle 7 (Rattenhaar)

| | |
|---|---|
| Präparat von Beispiel 7 [erfindungsgemäss, oxidatives Verfahren] | gelbe Verfärbung in 1 bis 2 Minuten |
| idem, + 10 mg/ml Harnstoff | gelbe Verfärbung, 186-206 Minuten |
| Salpetersäure 33,4% | gelbe Verfärbung, 210-230 Minuten |
| Salpetersäure 33,4% + 3 mg/ml $NaNO_2$* [erfindungsgemäss, Mischverfahren] | gelbe Verfärbung in 1 bis 2 Minuten |

* Zum Vergleich; in Tabelle 2A bereits angeführt.

Tabelle 8
(menschliche Haut, Unterseite des Vorderarms)

| 2 Minuten Einwirkungsdauer | sofort danach | nach 1 Stunde | nach 2¾ Stunden | nach 7 Stunden |
|---|---|---|---|---|
| Präparat von Beispiel 7 [erfindungsgemäss, oxidatives Verfahren] | brauner Fleck | der braune Fleck bleibt bestehen | | |
| idem, + 10 mg/ml Harnstoff | keine Reaktion | deutlicher rosa Fleck (Erythem) | deutlich rosa, aber heller | keine Spuren mehr |
| Salpetersäure 33,4% | keine Reaktion | deutlicher rosa Fleck | rosa Fleck, knapp sichtbar | keine Spuren mehr |
| Salpetersäure 33,4% + 8,9 mg/ml Cd(NO₂)₂ [erfindungsgemäss, Mischverfahren] | brauner Fleck | der braune Fleck bleibt bestehen[1] | | |

Auch nach 48 Stunden praktisch unverändert
Zum Vergleich; in Tabelle 2B bereits angeführt.

Das erfindungsgemässe Präparat stellt eine klare, farblose Flüssigkeit dar. Wird es in offenem Gefäss bei Raumtemperatur aufbewahrt, fallen die biologischen Teste nach weniger als zwei Wochen negativ aus, offensichtlich infolge der Zersetzung der salpetrigen Säure. Eine praktisch unbegrenzte Haltbarkeit kann durch Aufbewahrung zwischen 0 und 10 °C, z. B. im Kühlschrank, vorzugsweise bei etwa 4 °C und in locker verschlossenem Gefäss, erreicht werden.

Mit der erwähnten Haltbarkeit soll nicht etwa eine zahlenmässige Konstanz des Gehaltes an Salpetersäure und Nitrit und noch weniger des Gehaltes an anderen Komponenten gemeint sein: ausschlaggebend für die in der Kosmetik und der Klinik nachgewiesene Wirkung ist primär die erfindungsgemässe Zusammensetzung in bezug auf Salpetersäure und Nitrit. Sofern diese Komponenten in den angegebenen Bereichen vorliegen und welche Veränderungen ihr Gehalt im Laufe der Zeit innerhalb der angegebenen Bereiche auch erfahren mag, ist die Wirkung des Präparates gewährleistet.

Es ist auch ohne weiteres möglich, das Präparat in Form von zwei Ampullen darzubieten, deren erste eine Natriumnitritlösung, die zweite eine Lösung der anderen Komponenten oder deren erste die Salpetersäure, die zweite Milchsäure, Äthanol und die weiteren Komponenten enthält, und den Inhalt der beiden Ampullen vor der Anwendung miteinander zu vermischen.

Das neue Präparat ist im Tierversuch (Kaninchen) auf seine lokale Verträglichkeit geprüft worden. Es bewirkte keine nennenswerte Reaktion ausserhalb der Anwendungsstelle; an den (vorher rasierten) behandelten Hautstellen wachsen die Haare wieder nach wie bei den unbehandelten Kontrolltieren. Zudem konnten dabei überhaupt keine systemischen Wirkungen festgestellt werden.

Das Präparat soll einerseits in der Kosmetik, andererseits in der Medizin angewendet werden. Der kosmetische Indikationsbereich erstreckt sich auf alle gutartigen, kosmetisch störenden Hautveränderungen, sofern diese oberflächlich sind und eine bestimmte Grösse nicht überschreiten. Im Bereich der Kosmetik dürfen nur solche Fachkräfte das Präparat anwenden, welche vorher in einem besonderen Schulungskurs in seine Anwendungsweise und seine Wirkungsweise eingeführt worden sind.

Die Hauptanwendungsgebiete in der Medizin sind geeignete Indikationen in der Dermatologie, der Onkologie, der Urologie, der Ophthalmologie und der Gynäkologie. Das Präparat darf nur äusserlich und durch den Arzt oder auf seine Anweisung hin und unter seiner Kontrolle durch ausgebildete Medizinalpersonen angewendet werden, keinesfalls durch den Patienten selbst.

Die zur Zeit gebräuchlichen Methoden zur Behandlung der verschiedenen Hauttumoren, namentlich die Elektrokauterisation, die Kryochirurgie sowie die Strahlenbehandlung und die Chemotherapie oder die plastische Chirurgie, sind nicht immer voll befriedigend. Malignome oder als solche verdächtige Tumoren werden weit im gesunden Gewebe herausoperiert, miest nach vorheriger Probeexzision. Ein solches Vorgehen und etwa notwendige plastisch-chirurgische Massnahmen sind besonders in Augennähe oder im Nasenbereich schwierig oder gar bei multiplen Läsionen und bei Rezidiven, z. B. in vorbestrahltem Gebiet, nicht möglich. Die Methode von Mohs, die nach vorheriger Behandlung mit Trichloressigsäure und Zinkchlorid eine sukzessive Entfernung des pathologischen Gewebes und jeweils anschliessende histologische Kontrolle erlaubt, hat sich vor allem wegen der erheblichen Schmerzhaftigkeit nicht durchgesetzt. Demgegenüber kann das neue Präparat auch dann noch eingesetzt werden, wenn die anderen therapeutischen Möglichkeiten, namentlich Strahlenbehandlung und chirurgische Eingriffe, nicht oder nicht mehr angewendet werden können.

In der Tat führt das Präparat bei lokaler Anwendung zu einer unmittelbaren intravitalen Fixation des Gewebes, mit dem es in Berührung kommt. Sein Wirkungsbereich ist streng auf die behandelte Stelle begrenzt. Die unmittelbare Wirkung äussert sich in einer weiss-gelblich bis grauen Verfärbung der behandelten Stelle. Das so devitalisierte Gewebestück trocknet ein und verfärbt sich mit zunehmender Mumifizierung dunkelbraun. Der mumifizierte Schorf löst sich nach 2 bis 5 Wochen spontan ab. Die Abheilung erfolgt dank den im Präparat enthaltenen antiseptisch und heilungsfördernd wirkenden Metallionen ohne Komplikationen, namentlich ohne sekundäre Infektion, rasch und ohne hässliche Narben zu hinterlassen; infolgedessen bleiben allfällige Gewebezerrungen ebenfalls aus.

Die erwähnte intravitale Fixation wurde im Laboratoire des Recherches sur les Tumeurs de la Peau humaine, Département de Dermatologie der Fondation Adolphe de Rothschild, Paris, mit dem Präparat von Beispiel 7 elektronenmikroskopisch untersucht; dabei konnte gezeigt werden, dass sie sehr rasch erfolgt und von hoher Qualität ist. Die Strukturen des Stratum corneum und des Stratum malphighii bleiben dabei völlig erhalten, einschliesslich so empfindlicher Organellen wie der Desmosomen und Keratinosomen. Diese enthalten lytische Enzyme, die, im Falle einer verzögert einsetzenden Fixierung, zu deren teilweisen Verdauung führen würden.

Es scheint, dass das Präparat eine spezifische Wirkung auf hyperplastisches und metaplastisches ausübt. Eine spezifische antikanzeröse Wirkung des Präparates oder eine besondere Empfindlichkeit der Krebszellen gegenüber dem Präparat konnten bisher nicht nachgewiesen werden. Die scheinbare Spezifität dürfte auf eine Besonderheit des Tumorgewebes zurückgehen, das nämlich lockerer und weniger dicht und solide ist als normales, gesundes Gewebe. Der lockerere Verband der Tumorzellen kann eine raschere und leichtere Penetration der Lösung als der festere Zellverband des gesunden Gewebes zulassen; dieses und insbesondere das subkutane Collagen dürften wie ein Barriere wirken, welche ein Übergreifen des Präparates verhindert. So kann erklärt werden, dass die gesunden Zellen der Umgebung nicht oder nur in geringem Masse geschädigt werden. Die reparative Fähigkeit des umgebenden gesunden Gewebes bleibt nicht nur erhalten, sondern führt durch Phagozytose und Plasmazell-Reaktion in Verbindung mit einer Proliferation mesenchymaler Zellen zu einer raschen Regeneration und zum Ersatz des entstandenen Gewebedefektes.

Offenbar spielen die Metallionen eine wichtige Rolle inbezug auf die Penetrationsgeschwindigkeit und die Eindringtiefe des Präparates in die Haut; über diese Parameter, eventuell auch direkt, dürften sie die Reaktion der betupften Stelle und jene des umliegenden Gewebes und damit auch die Mumifikation bzw. die Abheilgeschwindigkeit mindestens zum Teil beeinflussen.

Die Indikationen des erfindungsgemässen Präparates sind oberflächliche oder von aussen zugängliche, benigne und prämaligne Veränderungen der Haut und der Schleimhäute, z.B.:

– Basaliome, besonders bei Rezidiven im Narbenbereich nach Bestrahlungen oder chirurgischen Eingriffen, in Augennähe und an der Nase oder den Ohren und bei multiplem Befall;

– epidermale Naevi, Fibrome der Haut und der Schleimhäute;

– senile Keratosen, Lentigo senilis, Lentigo praemaligna;

– seborrhoische Warzen (Verrucae seniles) und juvenile Warzen (Verrucae planae);

– Verrucae vulgares und Condylomata acuminata.

Das Präparat muss streng lokal, durch topisches Auftragen oder andere Formen der Lokalbehandlung, auf bzw. in die pathologisch veränderten Gewebsteile, deren Entfernung gewünscht wird, appliziert werden. Von der Mitte der Läsion ausgehend zur Peripherie hin werden ein bis mehrere Tröpfchen der Lösung verteilt und durch leichtes Sticheln in die Läsion eingearbeitet. Diese besondere Art der lokalen Anwendung spielt für den Erfolg der Behandlung eine beträchtliche Rolle; sie bildet einen integrierenden Bestandteil der Erfindung.

Als Applikator haben sich spitze Holzstäbchen, z.B. Zahnstocher, und dünne poröse Kunststoffstäbchen oder Filzschreiberspitzen, u.a. aus Polyäthylen oder Polypropylen, besonders bewährt. Wie bereits erwähnt wurde, kann das Präparat in Form zweier Ampullen verschiedenen Inhalts vorliegen; die genannten Applikatoren ermöglichen nun eine zweite, ebenso einfache Ausführung dieses Prinzips. Man kann nämlich die porösen Stäbchen in eine Nitritlösung eintauchen und dann vorsichtig trocknen; kurz vor der Anwendung taucht man die mit dem Nitrit imprägnierten Stäbchen in die Lösung der übrigen Komponenten ein.

Auf diese Weise wurden Zahnstocher aus Holz und angespitzte Kunststoffstäbchen, z.B. aus Polypropylen, einzeln oder zusammengebündelt, in eine 13%ige (Gewicht/Gewicht) Cadmiumnitritlösung eingetaucht und getrocknet. Mit den imprägnierten Stäbchen wurde eine 33,4%ige (Gewicht/Gewicht; 6,2 normal) Salpetersäurelösung gerührt und sodann dem in vitro-Test mit Rattenhaar unterworfen. Das Haar färbte sich gelb in weniger als 1 bis 2 Minuten; die Salpetersäurelösung enthielt also genügend Nitrit, um der geforderten Zusammensetzung zu entsprechen. Dieselben Stäbchen konnten dann in die nitrithaltige Lösung getränkt und als Applikator, zum Auftragen dieser frisch hergestellten Lösung auf die Läsion, benutzt werden.

Bei dieser Ausführungsform geht man mit Vorteil folgendermassen vor: Eine Lösung eines in wässriger Salpetersäure löslichen Metallnitrites, beispielsweise eine wässrige 10%ige Natriumnitritlösung, wird in ein Gefäss, z.B. eine Petri-Schale, vorgelegt, in dem die Eintauchtiefe festgelegt werden kann. Die Applikatorstäbchen werden, beispielsweise 1 cm tief und während 15 Minuten, in die Nitritlösung senkrecht eingetaucht und danach herausgezogen und in senkrechter Haltung trocknen gelassen.

Durch den in vitro-Test mit dem Rattenhaar konnte gezeigt werden, dass derart imprägnierte Applikatorstäbchen – ob aus Holz, einem harten, im landläufigen Sinne nicht porösen Kunststoff oder einem stark porösen Kunststoff – den Zweck erfüllen; werden die Stäbchen nämlich vier Tage später während 15 Sekunden in 0,3 ml einer 6,2 normalen Salpetersäurelösung eingetaucht, so weist dann diese Lösung den erfindungsgemässen Nitritgehalt auf (Tabelle 9).

Tabelle 9
(Rattenhaar)

| Applikatormaterial | gelbe Verfärbung (in Minuten)* |
|---|---|
| unbehandeltes Holz | 207-297 |
| $NaNO_3$-behandeltes Holz (Kontrolle) | 119-204 |
| $NaNO_2$-behandeltes Holz | 4-6 |
| unbehandelter nicht poröser Kunststoff | 293-383 |
| $NaNO_3$-behandelter nicht poröser Kunststoff (Kontrolle) | 95-170 |
| $NaNO_2$-behandelter nicht poröser Kunststoff | 4-5 |
| unbehandelter poröser Kunststoff | 180-270 |
| $NaNO_2$-behandelter poröser Kunststoff | 1-3 |

* Zeitpunkt der letzten negativen Beobachtung – Zeitpunkt der ersten positiven Beobachtung.

Bei der Dosierung und der Anwendungsdauer des Präparates müssen die Lokalisation, die Grösse und Dicke der Hautläsion und der Grad ihrer Verhornung berücksichtigt werden. Meistens werden zur Behandlung 0,05 bis 0,1 ml der Lösung benötigt. Hautveränderungen von mehr als 10 mm Durchmesser sollten erst dann behandelt werden, wenn Gewähr besteht, dass nur oberflächliche Hautanteile betroffen sind. Die Zahl der gleichzeitig behandelten Läsionen sollte 4 bis 5 und deren Gesamtfläche 5 cm² nicht überschreiten. Mehr als 0,25 ml sollte während einer Sitzung nicht verwendet werden. Bei unbefriedigender Mumufizierung kann nach 3 bis 4 Tagen eine zweite Behandlung erfolgen. Bei grösserer Anzahl von Hautläsionen ist die Behandlung in mehreren Sitzungen in Abständen von ca. 4 Wochen durchzuführen.

Es empfiehlt sich, zunächst nur einige Minuten zu behandeln, während kurzer Zeit die Reaktion abzuwarten und die Behandlung dann je nach der Intensität der Reaktion fortzusetzen, bis die lokale Verfärbung voll ausgeprägt ist und eine leichte Schrumpfung der Läsion beginnt. Die Verfärbung der Läsion geht unterschiedlich rasch vor sich. Während Basaliome eine rasche Gelbfärbung entwickeln, benötigen Keratosen und Fibrome eine längere Behandlungszeit. Die rasche Reaktion der Basaliome ist so auffallend, dass sie als differentialdiagnostischer Hinweis gewertet werden und als Basis für die Differentialdiagnose der Basaliome dienen kann. Nach offensichtlicher Abheilung eines Basalioms können so, durch weitere prophylaktische Anwendung des Präparates, eventuelle Basaliomreste entdeckt und nachbehandelt werden.

Die durch die Behandlung erfolgende Devitalisierung der veränderten Hautanteile ist im allgemeinen schmerzlos; gelegentlich wird ein leichtes, einige Minuten anhaltendes Brennen verspürt. Eine leichte Rötung der Umgebung der behandelten Hautstelle ist normal und bedarf keiner besonderen Behandlung. Bei stärkerer Umgebungsreaktion oder Juckreiz kann eine Steroidcrème oder eine anästhesierende Salbe lokal aufgetragen werden. Der Patient kann sich an dem der Behandlung folgenden Tag wieder waschen, baden oder duschen. Das Präparat wird nicht resorbiert und besitzt keine systemischen Wirkungen. Sensibilisierungen oder allergische Reaktionen wurden nicht beobachtet.

Die Vorteile des erfindungsgemässen Präparates und der auf seiner Verwendung beruhenden intravitalen Fixationsmethode können wie folgt zusammengefasst werden:

1. Die Behandlung ist auf die pathologisch veränderten Gewebeteile begrenzt, die entfernt werden sollen.

2. Der sich ergebende Gewebedefekt kann auf das notwendige Minimum beschränkt werden.

3. Die zurückbleibende Narbe ist meist unauffällig.

4. Das umgebende Gewebe wird nicht geschädigt.

5. Die Behandlung ist in der Regel schmerzlos.

6. Die Behandlung kann ambulant durchgeführt werden; der Patient ist anschliessend in seiner Aktivität nicht eingeschränkt.

7. Die Behandlung ist besonders von Vorteil, wenn multiple Läsionen vorhanden sind, oder vorangegangene Operationen oder Bestrahlungen eine erneute chirurgische Behandlung oder Bestrahlung ausschliessen.

In den nachfolgenden Beispielen erfolgte die Aufbewahrung der Präparate, wenn nicht anderes angegeben wird, bei Raumtemperatur und unter lockerem Verschluss.

Bevor ein Präparat für die Anwendung beim Menschen freigegeben wird, soll sich der Hersteller durch eine Analyse vergewissern, dass die Zusammensetzung und insbesondere der Nitritgehalt die erfindungsgemässen Anforderungen erfüllen. Wie den Beispielen entnommen werden kann, entspricht der anfänglich erhaltene Nitritgehalt nicht immer dem erfindungsgemäss verlangten Mindestwert, in welchem Fall weiteres Nitrit, beispielsweise Natriumnitrit, hinzugefügt werden soll.

Beispiel 1

750 mg Natriumnitrit werden in einen Messkolben zu 250 ml gegeben und mit 100 ml Wasser übergossen. Die Kristalle lösen sich auf; die Temperatur der Lösung beträgt 22,5 °C. Dann gibt man 128,5 ml 65%ige Salpetersäure zu; die Temperatur steigt auf ca. 40 °C. Die Lösung verfärbt sich hellgelb und entwickelt nitrose Gase. Man kühlt mit einem Wasserbad auf ca. 25 °C ab und füllt mit Wasser auf 250 ml auf.

Proben zu 10 ml des erhaltenen Präparats wurden in Kunststoff-Fläschchen zu 10 ml, mit Schraubdeckel, abgefüllt, um ihren Nitrat- und Nitritgehalt nach verschiedenen Zeitabständen zu bestimmen. Ein Teil der Fläschchen wurde verschlossen, der andere Teil offen bei Zimmertemperatur stehengelassen.

Verschlossene Proben

| Alter des Präparats | 70 Min. | 1 Tag | 7 Tage | |
|---|---|---|---|---|
| Nitratgehalt | 502 | 499 | 458 | mg/ml |
| Nitritgehalt | 1,03 | 1,26 | 0,59 mg/ml | |

Offene Proben

| Alter des Präparats | 165 Min. | 1 Tag | 7 Tage | |
|---|---|---|---|---|
| Nitratgehalt | 518 | 485 | 493 | mg/ml |
| Nitritgehalt | 1,10 | 0,25 | 0,003 mg/ml | |

Beispiel 2

0,077 g Kupfer(II)-nitrat-trihydrat, 8,64 g Cadmiumnitrat-tetrahydrat, 118,5 g Oxalsäure-dihydrat, 26,5 ml 90%ige Milchwsäure, 0,85 ml Brenztraubensäure, 80,1 ml Eisessig und 500 ml Wasser von ca. 20 °C werden miteinander vermischt. Beim Rühren gehen die Kristalle allmählich in Lösung.

Nach einer halben Stunde werden 1014 ml 65%ige Salpetersäure zugegeben. Das Gemisch erwärmt sich auf ca. 45 °C. Es wird mit 200 ml Wasser verdünnt und während 18 Stunden gerührt; dabei kühlt es sich allmählich auf 22 °C ab. Dann wird es durch Zugabe von Wasser auf 2000 ml verdünnt.

Die Analyse des 5 Monate alten Präparates ergab folgende Werte:

| Spezifisches Gewicht | 125 | mg/ml |
|---|---|---|
| Säureäquivalent | 9,15 | mmol/ml |
| Trockengewicht* | 779 | mg/ml |
| Gehalt an $Cd^{2+}$ | 1,62 | mg/ml |
| Gehalt an $Cu^{2+}$ | 0,012 | mg/ml |
| Oxalatgehalt | 33 | mg/ml |
| Lactatgehalt | 6,8 | mg/ml |
| Nitratgehalt | 464 | mg/ml |
| Nitritgehalt | 0,15 | mg/ml |
| Essigsäuregehalt | 42 | mg/ml |

* jeweils nach Neutralisation und Lyophilisation 6 Monate nach der Herstellung betrug das Säureäquivalent 8,88 mmol/ml, das Trockengewicht 755 mg/ml.

9 Monate nach der Herstellung: Nitratgehalt 442 mg/ml
10 Monate nach der Herstellung: Nitritgehalt 0,015 mg/ml
15 Monate nach der Herstellung: Nitritgehalt 0,018 mg/ml

**Beispiel 3**

0,086 g Kupfer(II)-nitrat-trihydrat, 113,5 g Oxalsäure-dihydrat, 41,1 ml 90%ige Milchsäure, 34,4 ml Eisessig und 0,85 ml Brenztraubensäure werden in 600 ml Wasser von ca. 20°C gelöst. Innerhalb von 10 Minuten werden 982 ml 65%ige Salpetersäure zugegeben. Das Gemisch erwärmt sich auf ca. 30 bis 35°C. Nach 2 Stunden wird es mit soviel Wasser verdünnt, dass sein Volumen 2000 ml beträgt.

Die Analysenergebnisse stammen von einem 3½ Monate alten Präparat. Das Präparat wurde 13 Monate nach seiner Herstellung erneut analysiert; inzwischen hatte es sich grün verfärbt und entliess braune Dämpfe ($NO_2$) beim Abnehmen des Verschlusses.

Datum der Herstellung 13. Juni 1978

| Datum der Bestimmung | 25.10.78 | 23.8.79 | |
|---|---|---|---|
| spezifisches Gewicht | 1246 | 1237 | mg/ml |
| Säureäquivalent | 8,66 | 10,06 | mmol/ml |
| Trockengewicht | 738 | 840 | mg/ml |
| $Cu^{2+}$-Gehalt | 0,015 | 0,014 | mg/ml |
| Oxalatgehalt | 34 | 46 | mg/ml |
| Lactatgehalt | 11 | ⟨0,02 | mg/ml |
| Nitratgehalt | 381 | 415 | mg/ml |
| Nitritgehalt | 0,0013 | 4,15 | mg/ml |
| Essigsäuregehalt | 21 | 22 | mg/ml |

**Beispiel 4**

124,4 g Oxalsäure-dihydrat, 27,9 ml 90%ige Milchsäure, 0,9 ml Brenztraubensäure, 84,1 ml Eisessig und ca. 500 ml Wasser von 20°C werden miteinander vermischt. Unter ständigem Rühren werden innerhalb von 10 Minuten 1065 ml 65%ige Salpetersäure zugegeben. Die Temperatur steigt dabei auf 30°C. Die Lösung ist farblos und klar; sie wird über Nacht bei Zimmertemperatur stehengelassen. Dabei bilden sich grosse Kristalle. Das Gemisch wird auf 25°C erwärmt und gerührt; die Kristalle lösen sich allmählich auf. Dann wird Wasser bis zu einem Volumen von 2000 ml hinzugefügt. Dabei bilden sich kleine farblose Kristalle. Das Gemisch wird eine Woche bei Zimmertemperatur stehengelassen. Die überstehende Lösung (Lösung A, siehe unten) ist klar und farblos. In einem Messkolben werden 8,843 g Zinknitrathexahydrat in Wasser gelöst und die Lösung auf 100 ml verdünnt. 5 ml dieser Zinknitrat-Lösung werden mit soviel der oben erwähnten Lösung A vermischt, dass das Volumen 100 ml beträgt.

Die Analyse des Präparates sofort nach seiner Herstellung ergab folgende Werte:

| spezifisches Gewicht | 1255 | mg/ml |
|---|---|---|
| Säureäquivalent | 9,13 | mmol/ml |
| Trockengewicht | 800 | mg/ml |
| Gehalt an $Zn^{2+}$ | 1,00 | mg/ml |
| Oxalatgehalt | 34 | mg/ml |
| Lactatgehalt | 12 | mg/ml |
| Nitratgehalt | 483 | mg/ml |
| Nitritgehalt | 0,0009 | mg/ml |
| Essigsäuregehalt | 26 | mg/ml |

6 Monate nach der Herstellung betrug der Nitritgehalt nur noch ca. 0,0001 mg/ml. Um das Präparat auf den Mindestgehalt von 0,01 mg/ml zu bringen, wurden 10 mg Natriumnitrit in 100 ml der Endlösung aufgelöst; dabei entwickelte sich sofort nitroses Gas, die Lösung blieb farblos. Der Lösung wurde eine Probe entnommen, sofort mit Wasser verdünnt und eine halbe Stunde später analysiert. Der Nitritgehalt, umgerechnet auf die unverdünnte Lösung, betrug 0,019 mg/ml (d.h. 30%, bezogen auf das zugesetzte Natriumnitrit).

**Beispiel 5**

Zu einem Gemisch aus 4,812 g Silbernitrat, 59,867 g Oxalsäure-dihydrat, 40 ml Eisessig und 13,23 ml 90%iger Milchsäure werden 300 ml Wasser von 20°C gegeben. Die Kristalle lösen sich teilweise auf. Innert 5 Minuten werden unter ständigem Rühren 507 ml 65%ige Salpetersäure zugetropft. Dabei steigt die Temperatur auf 31°C. 15 Minuten nach der Zugabe der Salpetersäure sind alle Kristalle gelöst; die Lösung ist klar und farblos. Sie wird mit Wasser verdünnt, bis ihr Volumen 1000 ml beträgt, und anschliessend bei Zimmertemperatur stehengelassen. Im Verlauf der nächsten drei Tage bilden sich grosse farblose Kristalle, welche durch Abfiltrieren entfernt werden.

Die Analyse des Präparates sofort nach seiner Herstellung (26. Januar 1979) ergab folgende Werte:

| spezifisches Gewicht | 1255 | mg/ml |
|---|---|---|
| Säureäquivalent | 8,31 | mmol/ml |
| Trockengewicht | 707 | mg/ml |
| Gehalt an $Ag^+$ | 3,17 | mg/ml |
| Oxalatgehalt | 34 | mg/ml |
| Lactatgehalt | 12 | mg/ml |
| Nitratgehalt | 420 | mg/ml |
| Nitritgehalt | 0,0037 | mg/ml |
| Essigsäuregehalt | 32 | mg/ml |

Im Verlauf eines halben Jahres liess sich eine geringe Grünfärbung wahrnehmen; 217 Tage nach der Herstellung ergab eine neue Bestimmung einen Nitritgehalt von 1,32 mg/ml.

### Beispiel 6

285,2 g L-Weinsäure und 8,330 g Zinknitrathexahydrat werden in einem Messkolben zu 2000 ml vorgelegt und mit ca. 600 ml Wasser übergossen. Ein Teil der Kristalle löst sich. Dann werden innerhalb einer Viertelstunde 1013,2 ml 65%ige Salpetersäure zugegeben; durch Kühlen in einem Wasserbad wird dafür gesorgt, dass die Temperatur des Kolbeninhalts nicht über 25°C steigt. Schliesslich wird mit Wasser auf 2000 ml aufgefüllt. Mit Hilfe eines Magnetrühres wird solange gerührt, bis sich alle Kristalle gelöst haben; die Lösung ist klar und farblos. Im Verlaufe eines Jahres, im verschlossenen Messkolben aufbewahrt, verfärbt sie sich grün und entwickelt beim Abnehmen des Verschlusses braune Dämpfe ($NO_2$). Ferner scheiden sich allmählich kleine farblose Kristalle ab.

Datum der Herstellung 22. Mai 1978

| Datum der Bestimmung | 31.5.78 | 20.6.78 | 16.8.79 | |
|---|---|---|---|---|
| spezifisches Gewicht | 1296 | — | 1238 | mg/ml |
| Säureäquivalent | 9,47 | 9,42 | 7,79 | mmol/ml |
| Trockengewicht | 884 | 844 | 667 | mg/ml |
| $Zn^{2+}$-Gehalt | 1,07 | — | 0,83 | mg/ml |
| Oxalatgehalt | — | 0 | 39 | mg/ml |
| Nitratgehalt | 530 | 523 | 369 | mg/ml |
| Nitritgehalt | — | — | 4,45 | mg/ml |

### Beispiel 7

In 1000 ml 65%iger Salpetersäure werden 1,5 g metallisches Cadmium und 22,5 g metallisches Kupfer aufgelöst. Unter kräftigem Rühren und gelindem Kühlen mit Hilfe eines Wasserbads von ca. 20°C werden innert 30 Minuten 230 g 80%ige Milchsäure zugetropft. Anschliessend werden 50 ml 40%iges Äthanol innert 20 Minuten zugetropft. Während der Äthanolzugabe steigt die Temperatur des Reaktionsgemisches auf ca. 35 bis 40°C. Kurz nach der Zugabe des Äthanols entsteht ein hellblauer Niederschlag. 3 Stunden nach der Äthanolzugabe werden 1 g Oxalsäuredihydrat, 10 Minuten später 1 g Maleinsäure und weitere 10 Minuten später 15 ml Brenztraubensäure zugegeben. Das Wasserbad wird jetzt auf 35 bis 40°C eingestellt. Sobald auch das Reaktionsgemisch diesen Temperaturbereich erreicht hat, werden 20 ml Eisessig innert 10 Minuten zugetropft. Das Gemisch wird eine Woche lang unter ständigem Rühren bei 35 bis 40°C gehalten; nachher wird es bei Zimmertemperatur aufbewahrt. Dabei soll es nur leicht verschlossen sein, weil sich auch jetzt noch Blasen bilden. Durch gelegentliches Schütteln wird der Austritt der Gasblasen aus der Flüssigkeit erleichtert und damit der Reifungsprozess gefördert. Der Reifungsprozess gilt als abgeschlossen, wenn die Flüssigkeit klar und farblos geworden ist und sich keine Gasblasen mehr bilden. Dann wird die überstehende Flüssigkeit vom Niederschlag dekantiert; nur sie findet als Heilmittel Verwendung.

### Beispiel 8

Zu 1798 ml 65%iger Salpetersäure werden unter Rühren 137 ml Wasser, 171 g Kupfer(II)-nitrat-trihydrat und 8,23 g Cadmiumnitrat-tetrahydrat gegeben. Die Salze lösen sich auf. Unmittelbar anschliessend werden innert 30 Minuten 338 ml 90%ige Milchsäure zugetropft. Innerhalb der nächsten 30 Minuten wird ein Gemisch aus 47 ml absolutem Äthanol und 56 ml Wasser zugetropft. 15 Minuten nach Beendigung der Äthanolzugabe setzt eine Gasentwicklung ein, und die Lösung erwärmt sich. Sie wird, sobald sie die Temperatur von 40°C erreicht hat, äusserlich mit kaltem Wasser und Eis gekühlt; dabei fällt die Temperatur allmählich auf ca. 30°C. 3 Stunden nach der Äthanolzugabe werden innert 10 Minuten 2 g Oxalsäure-dihydrat, 2 g Maleinsäure und 30 ml Brenztraubensäure zugegeben, nach einer Pause von 10 Minuten auch 40 ml Eisessig. Die Temperatur wird ständig auf ca. 30°C gehalten. Das Gemisch wird weitere 24 Stunden gerührt. Anschliessend wird es bei Zimmertemperatur stehengelassen und nach Beispiel 7 weiterverarbeitet.

### Beispiel 9

Zu 18,7 ml Wasser werden 40,9 g 90%ige Milchsäure, 5 ml absolutes Äthanol, 3 ml Brenztraubensäure, 0,2 g Oxalsäure-dihydrat, 0,2 g Maleinsäure, 4 ml Eisessig, 0,823 g Cadmiumnitrat-tetrahydrat und 17,11 g Kupfer(II)-nitrat-trihydrat gegeben. Das Gemisch wird gerührt und auf 50°C erwärmt. Die Kristalle lösen sich unter diesen Bedingungen zum grössten Teil auf. Innerhalb einer Stunde werden 180 ml 65%ige Salpetersäure zugetropft. Es entsteht eine klare, dunkelblaue Lösung. Sie wird auf ca. 20°C abgekühlt und 3 Tage stehengelassen. Während dieser Zeit wird keine Reaktion beobachtet. Am vierten Tag nach Herstellungsbeginn wird die Lösung auf 50°C erwärmt. Sie beginnt zu schäumen und entwickelt nitrose Gase. Die Temperatur steigt innert 10 Minuten auf 78°C, und es entsteht ein feinkörniger hellblauer Niederschlag. Das Gemisch wird mit kaltem Wasser auf ca. 50°C abgekühlt, weitere 8 Stunden unter Rühren bei dieser Temperatur gehalten und schliesslich bei Zimmertemperatur stehengelassen und nach Beispiel 7 weiterverarbeitet.

Beispiel 10

20,5 g 90%ige Milchsäure, 2,5 ml absolutes Äthanol, 1,5 ml Brenztraubensäure und 0,1 g Maleinsäure werden miteinander vermischt. Innert 30 Minuten werden bei 20 °C 90 ml 65%ige Salpetersäure zugetropft. Die Lösung bleibt klar und farblos. Nachher werden 0,1 g Oxalsäure-dihydrat, 2,0 ml Eisessig, 0,412 g Cadmiumnitrat-tetrahydrat, 8,55 g Kupfer(II)-nitrat-trihydrat und 9,4 ml Wasser zugegeben. Es entsteht eine tiefblaue Lösung. Sie wird eine Stunde lang bei 55 °C gerührt, ohne dass sich eine Reaktion zeigt. Anschliessend wird die Lösung 16 Stunden bei Zimmertemperatur stehengelassen. Beim nachfolgenden Erwärmen auf 50 °C beginnt die Entwicklung nitroser Gase und die Abscheidung eines feinkörnigen hellblauen Niederschlags. Das Gemisch wird 4 Stunden bei 55 °C gerührt und anschliessend bei Zimmertemperatur stehengelassen und nach Beispiel 7 weiterverarbeitet.

Analysenergebnisse

|  | Beispiel 7 | Beispiel 8 | Beispiel 9 | Beispiel 10 |  |
|---|---|---|---|---|---|
| Alter des Präparates | 385 Tage | 195 Tage | 24 Tage | 20 Tage |  |
| spezifisches Gewicht | 1236 | 1244 | 1268 | 1270 | mg/ml |
| Säureäquivalent | 8,38 | 8,64 | 9,21 | 9,46 | mmol/ml |
| Trockengewicht | 778 | 740 | 797 | 826 | mg/ml |
| Gehalt an $Cd^{2+}$ | 1,52 | 1,50 | 2,15 | 1,51 | mg/ml |
| Gehalt an $Cu^{2+}$ | 0,013 | 0,011 | 0,055 | 0,014 | mg/ml |
| Oxalatgehalt | 40 | 39 | 35 | 33 | mg/ml |
| Lactatgehalt | 4,7 | 14 | 20 | 37 | mg/ml |
| Nitratgehalt | 381 | 349 | 428 | 460 | mg/ml |
| Nitritgehalt | 0,099 | 0,10 | 0,28 | 0,68 | mg/ml |
| Essigsäuregehalt | 38 | 41 | 32 | 28 | mg/ml |

Beispiel 11

Ein wie im Beispiel 7 nach dem oxidativen Verfahren hergestelltes Präparat zeigte nach einjährigem Stehen unter lockerem Verschluss die folgenden Gehalte:

| spezifisches Gewicht | 1246 | mg/ml |
|---|---|---|
| Säureäquivalent | 8,62 | mmol/ml |
| Trockengewicht | 787 | mg/ml |
| $Cu^{2+}$-Gehalt | 0,011 | mg/ml |
| $Cd^{2+}$-Gehalt | 1,50 | mg/ml |
| Oxalatgehalt | 42 | mg/ml |
| Lactatgehalt | 12 | mg/ml |
| Nitratgehalt | 394 | mg/ml |
| Nitritgehalt | 0,179 | mg/ml |
| Essigsäuregehalt | 44 | mg/ml |

Eine Probe dieses Präparates wurde, ca. 15 Monate nach seiner Herstellung, in ein Kunststoff-Fläschchen mit Schraubdeckel abgefüllt und unter hermetischem Verschluss (Schraubdeckel satt zugedreht) bei Zimmertemperatur stehengelassen. Nach 3 Wochen hatte sich der Inhalt des Fläschchens grün verfärbt und entwickelte beim Abnehmen des Schraubdeckels reichlich Gasblasen; sie enthielten $NO_2$ (braun, Geruch). Durch Quetschen des Kunststoff-Fläschchens, Schütteln und Blasen konnte ein grosser Teil des in der Flüssigkeit gelösten Gases ausgetrieben werden. Schraubte man den Deckel jetzt wieder zu und schüttelte man das Fläschchen, so erhöhte sich der Druck im Innern, und beim Abnehmen des Deckels schäumte die Lösung unter Freisetzung von $NO_2$ auf. Die Nitrit-Bestimmung der im Fläschchen befindlichen Probe zeigte jetzt, sogar nach mehrmaligem Schütteln und Ausblasen des Gases, einen Gehalt von 25,93 mg/ml Nitrit, während die unter lockerem Verschluss gehaltene Hauptmenge des Präparates einen Nitritgehalt von 0,089 mg/ml besass. Sowohl der Fläschcheninhalt als auch die Hauptmenge des Präparats wurden einer zweiten Nitritbestimmung unterzogen, nachdem eine kleine Probe dieser beiden Flüssigkeiten 24 Stunden in einem offenen Gefäss gestanden hatten. Der Fläschcheninhalt besass nach 24-stündigem Stehen im offenen Gefäss noch 0,015 mg/ml Nitrit, die Hauptmenge noch 0,001 mg/ml Nitrit.

Die Veränderung, die der Fläschcheninhalt beim Stehen unter hermetischem Verschluss erlitten hat, ist darauf zurückzuführen, dass der hermetische Verschluss die sich ganz langsam entwickelnden Gase am Entweichen hindert und zu einer Druckerhöhung im Innern des Gefässes führt, die allem Anschein nach die oxidative Zersetzung der oxidierbaren Anteile beschleunigt, bis ein Gleichgewicht erreicht wird.

Entsprechende Veränderungen, begleitet von der Verfärbung nach blaugrün und der Entwicklung nitroser Gase, erleiden auch die durch das Mischverfahren hergestellten Präparate. Dies wurde u. a. an den Präparaten der Beispiele 3 und 6 beobachtet und durch analytische Bestimmungen belegt. Beide Präparate waren ursprünglich farblos und entwickelten in den ersten Monaten nach ihrer Herstellung keine Gasblasen. Das Präparat von Beispiel 3 besass dreieinhalb Monate nach seiner Herstellung einen Lactatgehalt von 11 mg/ml und einen Nitritgehalt von 0,0013 mg/ml. Etwa acht Monate nach seiner Herstellung wurde erstmals die gründe Verfärbung beobachtet. 10 Monate nach der ersten Bestimmung wurde das Präparat, nun deutlich grün gefärbt, erneut analysiert. Diesmal besass es 4,15 mg/ml Nitrit; Lactat konnte – im Gegensatz zu der ersten Bestimmung – überhaupt nicht mehr nachgewiesen werden. Signifikante, wenn auch weniger extreme Unterschiede wurden beim Säureäquivalent (Erhöhung), Trockengewicht (Erhöhung) und Oxalatgehalt (Erhöhung) beobachtet, während eine geringfügige Erhöhung des Nitratgehaltes nicht als signifikante Veränderung gilt.

Das Präparat von Beispiel 5, hergestellt aus Weinsäure, Zinknitrat und Salpetersäure, war ursprünglich ebenfalls farblos und zeigte keine Tendenz zur Gasentwicklung. Die Bestimmung, die einen Monat nach seiner Herstellung durchgeführt wurde, ergab die gleichen Werte wie die zehn Tage nach der Herstellung durchgeführte Bestimmung; bei der Untersuchung nach einem Monat wurde keine Oxalsäure nachgewiesen. Fünf Monate nach der Herstellung wurde ein etwas niedrigeres Säureäquivalent gefunden; zu jener Zeit war noch keine auffällige Verfärbung zu sehen. 15 Monate nach der Herstellung war das Präparat deutlich grün. Es enthielt jetzt 39 mg/ml Oxalat und 4,45 mg/ml Nitrit. Signifikante Veränderungen zeigten ferner Säureäquivalent (Abnahme), Trockengewicht (Abnahme) und Nitratgehalt (Abnahme).

Die geschilderten Veränderungen, erkennbar an der Verfärbung nach grün und der Freisetzung nitroser Gase, treten nicht immer gleich rasch auf. So erfolgte die Veränderung bei dem in Kunststoff-Fläschchen abgefüllten Präparat (Beispiel 7) innert weniger Wochen und führte zu einem Nitritgehalt, der vor dem Öffnen des unter hohem Innendruck befindlichen Fläschchens bedeutend höher als 25,93 mg/ml gewesen sein musste. Bei den beiden anderen Präparaten jedoch (Beispiele 3 und 6) war die Verfärbung erst nach mehreren Monaten erkennbar. Die Unterschiede, auch die verschieden hohen Nitritgehalte der verfärbten Lösungen, sind offensichtlich darauf zurückzuführen, dass das eine Präparat nur sehr wenig Luftraum im Innern des hermetisch verschlossenen Gefässes besass, während den beiden anderen Präparaten ein relativ grosses Gasvolumen zur Verfügung stand. Ferner scheint die Geschwindigkeit der Veränderung von der Dichtigkeit des Verschlusses abzuhängen. So beobachtet man beim Präparat von Beispiel 2 keine Verfärbung nach grün und keine Gasentwicklung, obwohl es in einem gleichen Gefäss aufbewahrt wurde wie die Präparate der Beispiele 3 und 6; es ist anzunehmen, dass der Glasstopfen über dem Präparat von Beispiel 2 nicht hermetisch verschloss.

Schliesslich ist auch zu beachten, dass der Oxalatgehalt sämtlicher Präparate etwa um 40 mg/ml liegt. Auch das ursprünglich oxalatfreie Präparat von Beispiel 6 zeigt einen Oxalatgehalt dieser Grössenordnung; es handelt sich hier vermutlich um den Gehalt der gesättigten Lösung.

Der Nitritgehalt einer Probe hängt nicht nur vom Verschluss des Gefässes ab und damit von der Bildungsgeschwindigkeit der nitrosen Gase ab, sondern auch von der Entweichgeschwindigkeit. Dies zeigt sich deutlich, wenn die Präparate der Beispiele 3, 6 und 7 (Fläschchen) einerseits und von Beispiel 1 andererseits verglichen werden. Dort liess man die nitritreichen Flüssigkeiten über Nacht in einem flachen Schälchen stehen, das in einem Exsikkator ohne Verschluss stand; die Dämpfe konnten ungehindert aus der Flüssigkeit austreten, den Exsikkator füllen und ihn allmählich durch seine Öffnung verlassen. Beim Beispiel 1 hingegen war lediglich der Deckel eines Fläschchens abgeschraubt; der Austritt der Gasmolekeln aus der Flüssigkeit war hier weniger leicht. Diese Unterschiede zeigen sich deutlich an den Nitritgehalten, die nach 24-stündigem Stehen gefunden werden. Bei den drei Flüssigkeiten, die in flachen Schälchen gestanden hatten, war er auf rund ein Tausendstel seiner ursprünglichen Höhe gefallen (von 4,15 auf 0,005; von 4,45 auf 0,004; von 25,93 auf 0,015), während er beim Beispiel 1 (im Fläschchen) nur auf ein Viertel sank und für das weitere Absinken eine Woche benötigte.

### Patentansprüche für die Vertragsstaaten BE, CH, DE, FR, GB, IT, LI, NL, SE

1. Präparat zur lokalen Behandlung von oberflächlichen Haut- und Schleimhautveränderungen benigner und prämaligner Art und Basaliomen, dadurch gekennzeichnet, dass es aus einer wässrigen Lösung besteht, welche

(1) Salpetersäure in einer Konzentration und Menge, welche der Lösung einen pH-Wert unterhalb von 1 und ein Säureäquivalent von 6 bis 10 Millimol/ml verleihen, und

(2) ein in wässriger Salpetersäure lösliches Metallnitrit oder salpetrige Säure in einer Menge, welche 0,01 bis 5 mg Nitrit per ml entspricht, enthält.

2. Präparat nach Anspruch 1, dadurch gekennzeichnet, dass die wässrige Lösung ausserdem mindestens eine durch Salpetersäure oxidierbare organische Carbonsäure enthält.

3. Präparat nach Anspruch 2, dadurch gekennzeichnet, dass besagte organische Carbonsäure eine aliphatische Hydroxisäure, Ketosäure oder ungesättigte Säure ist.

4. Präparat nach Anspruch 2 oder 3, dadurch gekennzeichnet, dass besagte organische Carbonsäure Oxalsäure oder Milchsäure ist.

5. Präparat nach Anspruch 1, dadurch gekennzeichnet, dass die wässrige Lösung ausserdem mindestens ein bei äusserlicher Anwendung nicht toxisches, in wässriger Salpetersäure bei einem pH unterhalb von 1 lösliches Metallsalz enthält.

6. Präparat nach Anspruch 5, dadurch gekennzeichnet, dass besagtes Metallsalz ein solches von Kupfer, Silber, Cadmium oder/und Zink ist.

7. Präparat nach Anspruch 2, dadurch gekennzeichnet, dass die wässrige Lösung ausserdem mindestens ein Metallsalz mit den in Anspruch 5 genannten Eigenschaften enthält.

8. Präparat nach Anspruch 4, dadurch gekennzeichnet, dass die wässrige Lösung ausserdem ein Kupfer- und/oder ein Cadmiumsalz mit den in Anspruch 5 genannten Eigenschaften enthält.

9. Präparat nach Anspruch 1, dadurch gekennzeichnet, dass die wässrige Lösung ausserdem eine keratolytisch wirkende organische Säure enthält.

10. Verfahren zur Herstellung des Präparates nach Anspruch 1, dadurch gekennzeichnet, dass man ein in wässriger Salpetersäure lösliches Metallnitrit in einer Menge, welche 0,01 bis 5 mg Nitrit ($NO_2^{\ominus}$) per ml der Endlösung entspricht, mit einer Salpetersäurelösung der Konzentration 6 bis 10 Millimol/ml oder mit einer konzentrierteren Salpetersäurelösung reagieren lässt und im letzteren Fall die erhaltene Lösung mit Wasser auf einen Gehalt von 6 bis 10 Millimol Salpetersäure per ml und einen pH-Wert unterhalb von 1 verdünnt.

11. Verfahren zur Herstellung des Präparates nach Anspruch 7 oder 8, dadurch gekennzeichnet, dass man besagte organische(n) Carbonsäure(n) und besagtes oder besagte Metallsalze in Wasser auflöst, die wässrige Lösung durch Zugabe einer konzentrierten Salpetersäurelösung und allfällige Verdünnung mit Wasser auf eine Salpetersäurekonzentration von 6 bis 10 Millimol/ml und einen pH-Wert unterhalb von 1 bringt und mit einem in wässriger Salpetersäure löslichen Metallnitrit in einer Menge, welche 0,01 bis 5 mg Nitrit per ml der Endlösung entspricht, reagieren lässt.

12. Verfahren zur Herstellung eines Präparates nach Ansprüchen 8 und 9, dadurch gekennzeichnet, dass man in beliebiger Reihenfolge Kupferionen, Cadmiumionen, konzentrierte Salpetersäure, Milchsäure oder einen Milchsäureniederalkylester, Oxalsäure oder einen Oxalsäuredi(niederalkyl)ester, Essigsäure oder einen Essigsäureniederalkylester und gegebenenfalls Äthanol miteinander vermischt und bei einer Temperatur von 20 bis 100 °C ausreagieren lässt, bis die Flüssigkeit klar und farblos geworden ist und einen pH-Wert unterhalb von 1 aufweist und sich praktisch keine Gasblasen mehr bilden, und die überstehende Flüssigkeit von dem entstandenen Bodenkörper, der verworfen wird, abtrennt.

13. Verfahren nach Anspruch 12, dadurch gekennzeichnet, dass man konzentrierte Salpetersäure, ein wasserlösliches Kupfer(II)-salz oder metallisches Kupfer mit oder ohne ein wasserlösliches Cadmiumsalz oder metallisches Cadmium miteinander auflöst bzw. reagieren lässt, die entstandene Lösung mit Milchsäure oder Milchsäureäthylester und eventuell mit wässrigem Äthanol versetzt und reagieren lässt, die gebildete Lösung mit Oxalsäure oder Oxalsäurediäthylester und hierauf mit Essigsäure oder Essigsäureäthylester versetzt und die Lösung ausreagieren lässt, bis die Flüssigkeit klar und farblos geworden ist und einen pH-Wert unterhalb von 1 aufweist und sich praktisch keine Gasblasen mehr bilden, und die überstehende Flüssigkeit von dem entstandenen Bodenkörper, der verworfen wird, abtrennt, wobei die Temperatur während der ganzen Herstellungsdauer auf Raumtemperatur bis leicht erhöhter Temperatur, vorzugsweise 20 bis 40 °C, durch Abkühlen oder Erwärmen gehalten wird.

14. Verfahren zur Herstellung des Präparates nach Anspruch 1 unmittelbar vor dessen Anwendung durch Kontaktieren eines in wässriger Salpetersäure löslichen Metallnitrits und einer wässrigen Salpetersäurelösung der Konzentration 6 bis 10 Millimol/ml und vom pH-Wert unterhalb von 1, dadurch gekennzeichnet, dass man ein als Applikator bestimmtes poröses, spitzes Stäbchen mit einem solchen Metallnitrit vorbehandelt oder imprägniert.

15. Mittel zur Ausführung des Verfahrens nach Anspruch 14, bestehend aus einem porösen, spitzen Applikatorstäbchen, welches mit einem in wässriger Salpetersäurelösung löslichen Metallnitrit in einer Menge von 0,01 bis 5 mg imprägniert ist.

## Patentansprüche für den Vertragsstaat AT

1. Verfahren zur Herstellung eines Präparates zur lokalen Behandlung von oberflächlichen Haut- und Schleimhautveränderungen benigner und prämaligner Art und Basaliomen, dadurch gekennzeichnet, dass man ein in wässriger Salpetersäure lösliches Metallnitirt in einer Menge, welche 0,01 bis 5 mg Nitrit ($NO_2-$) per ml der Endlösung entspricht, mit einer Salpetersäurelösung der Konzentration 6 bis 10 Millimol/ml oder mit einer konzentrierteren Salpetersäurelösung reagieren lässt und im letzteren Fall die erhaltene Lösung mit Wasser auf einen Gehalt von 6 bis 10 Millimol Salpetersäure per ml und einen pH-Wert unterhalb von 1 verdünnt.

2. Verfahren zur Herstellung eines Präparates nach Anspruch 1, welches ausserdem mindestens eine durch Salpetersäure oxidierbare organische Carbonsäure und mindestens ein bei äusserlicher Anwendung nicht toxisches, in wässriger Salpetersäure bei einem pH unterhalb von 1 lösliches Metallsalz enthält, dadurch gekennzeichnet, dass man besagte organische(n) Carbonsäure(n) und besagtes oder besagte Metallsalze in Wasser auflöst, die wässrige Lösung durch Zugabe einer konzentrierten Salpetersäurelösung und allfällige Verdünnung mit Wasser auf eine Salpetersäurekonzentration von 6 bis 10 Millimol/ml und einen pH-Wert unterhalb von 1 bringt und mit einem in wässriger Salpetersäure löslichen Metallnitrit in einer Menge, welche 0,01 bis 5 mg Nitrit per ml der Endlösung entspricht, reagieren lässt.

3. Verfahren nach Anspruch 2, dadurch gekennzeichnet, dass besagte organische Carbonsäure eine aliphatische Hydroxisäure, Ketosäure oder ungesättigte Säure ist.

4. Verfahren nach Anspruch 2 oder 3, dadurch gekennzeichnet, dass besagte organische Carbonsäure Oxalsäure oder Milchsäure ist.

5. Verfahren nach Anspruch 2, dadurch gekennzeichnet, dass besagtes Metallsalz ein solches von Kupfer, Silber, Cadmium oder/und Zink ist.

6. Verfahren nach Ansprüchen 4 und 5, dadurch gekennzeichnet, dass besagte organische Carbonsäure Oxalsäure oder Milchsäure und besagtes Metallsalz ein Kupfer- und/oder ein Cadmiumsalz ist.

7. Verfahren zur Herstellung eines Präparates nach Anspruch 1, welches ausserdem eine keratolytisch wirkende organische Säure enthält, gekennzeichnet durch die entsprechende Zugabe.

8. Verfahren nach Ansprüchen 6 und 7, dadurch gekennzeichnet, dass man in beliebiger Reihenfolge Kupferionen, Cadmiumionen, konzentrierte Salpetersäure, Milchsäure oder einen Milchsäureniederalkylester, Oxalsäure oder einen Oxalsäuredi(niederalkyl)ester, Essigsäure oder einen Essigsäureniederalkylester und gegebenenfalls Äthanol miteinander vermischt und bei einer Temperatur von 20 bis 100 °C ausreagieren lässt, bis die Flüssigkeit klar und farblos geworden ist und einen pH-Wert unterhalb von 1 aufweist und sich praktisch keine Gasblasen mehr bilden, und die überstehende Flüssigkeit von dem entstandenen Bodenkörper, der verworfen wird, abtrennt.

9. Verfahren nach Anspruch 8, dadurch gekennzeichnet, dass man konzentrierte Salpetersäure, ein wasserlösliches Kupfer(II)-salz oder metallisches Kupfer mit oder ohne ein wasserlösliches Cadmiumsalz oder metallisches Cadmium miteinander auflöst bzw. reagieren lässt, die entstandene Lösung mit Milchsäure oder Milchsäureäthylester und eventuell mit wässrigem Äthanol versetzt und reagieren lässt, die gebildete Lösung mit Oxalsäure oder Oxalsäurediäthylester und hierauf mit Essigsäure oder Essigsäureäthylester versetzt und die Lösung ausreagieren lässt, bis die Flüssigkeit klar und farblos geworden ist und einen pH-Wert unterhalb von 1 aufweist und sich praktisch keine Gasblasen mehr bilden, und die überstehende Flüssigkeit von dem entstandenen Bodenkörper, der verworfen wird, abtrennt, wobei die Temperatur während der ganzen Herstellungsdauer auf Raumtemperatur bis leicht erhöhter Temperatur, vorzugsweise 20 bis 40 °C, durch Abkühlen oder Erwärmen gehalten wird.

10. Verfahren zur Herstellung des Präparates nach Anspruch 1 unmittelbar vor dessen Anwendung durch Kontaktieren eines in wässriger Salpetersäure löslichen Metallnitrits und einer wässrigen Salpetersäurelösung der Konzentration 6 bis 10 Millimol/ml und vom pH-Wert unterhalb von 1, dadurch gekennzeichnet, dass man ein als Applikator bestimmtes poröses, spitzes Stäbchen mit einem solchen Metallnitrit vorbehandelt oder imprägniert.

11. Mittel zur Ausführung des Verfahrens nach Anspruch 10, bestehend aus einem porösen, spitzen Applikatorstäbchen, welches mit einem in wässriger Salpetersäurelösung löslichen Metallnitrit in einer Menge von 0,01 bis 5 mg imprägniert ist.

**Claims for the Contracting States BE, CH, DE, FR, GB, IT, LI, NL and SE**

1. Product for the topical treatment of superficial skin and mucosal lesions of benign and premalignant types and basalomas, characterized in that it is composed of an aqueous solution which contains

(1) nitric acid in a concentration and amound which confer on the solution a pH below 1 and an acid equivalent of 6 to 10 millimoles/ml, and

(2) a metal nitrite which is soluble in aqueous nitric acid, or nitrous acid, in an amount which corresponds to 0.01 to 5 mg of nitrite per ml.

2. Product according to Claim 1, characterized in that the aqueous solution additionally contains at least one organic carboxylic acid which is oxidizable by nitric acid.

3. Product according to Claim 2, characterized in that the said organic carboxylic acid is an aliphatic hydroxy acid, keto acid or unsaturated acid.

4. Product according to Claim 2 or 3, characterized in that the said organic carboxylic acid is oxalic acid or lactic acid.

5. Product according to Claim 1, characterized in that the aqueous solution additionally contains at least one metal salt which is non-toxic on external use and is soluble in aqueous nitric acid at a pH below 1.

6. Product according to Claim 5, characterized in that the said metal salt is one of copper, silver, cadmium and/or zinc.

7. Product according to Claim 2, characterized in that the aqueous solution additionally contains at least one metal salt having the properties mentioned in Claim 5.

8. Product according to Claim 4, characterized in that the aqueous solution additionally contains a copper and/or a cadmium salt having the properties mentioned in Claim 5.

9. Product according to Claim 1, characterized in that the aqueous solution additionally contains an organic acid having a keratolytic action.

10. Process for the preparation of the product according to Claim 1, characterized in that a metal nitrite which is soluble in aqueous nitric acid is allowed to react, in an amount which corresponds to 0.01 to 5 mg of nitrite ($NO_2^-$) per ml of the final solution, with a nitric acid solution of concentration 6 to 10 millimoles/ml, or with a concentrated nitric acid solution, and in the latter case the resulting solution is diluted with water to a content of 6 to 10 millimoles of nitric acid per ml and to a pH below 1.

11. Process for the preparation of the product according to Claim 7 or 8, characterized in that the said organic carboxylic acid(s) and the said metal salts(s) are dissolved in water, and the aqueous solution is adjusted by addition of a concentrated nitric acid solution and, where necessary, dilution with water to a nitric acid concentration of 6 to 10 millimoles/ml and a pH below 1, and is allowed to react with a metal nitrite which is soluble in aqueous nitric acid, in an amount which corresponds to 0.01 to 5 mg of nitrite per ml of the final solution.

12. Process for the preparation of a product according to Claims 8 and 9, characterized in that, in arbitrary sequence, copper ions, cadmium ions, concentrated nitric acid, lactic acid or a lower alkyl lactate, oxalic acid or a (di(lower alkyl) oxalate, acetic acid or a lower alkyl acetate, and, where appropriate, ethanol, are mixed together and allowed to react completely, at a temperature of 20 to 100°C, until the liquid has become clear and colourless and has a pH below 1, and virtually no more gas bubbles are formed, and the supernatant liquid is separated off from the resulting sediment, which is discarded.

13. Process according to Claim 12, characterized in that concentrated nitric acid, a water-soluble copper(II) salt or metallic copper, with or without a water-soluble cadmium salt or metallic cadmium, are together dissolved and allowed to react, the resulting solution is mixed and allowed to react with lactic acid or ethyl lactate and, possibly, with aqueous ethanol, the solution which has formed is mixed with oxalic acid or diethyl oxalate and then with acetic acid or ethyl acetate, and the solution is allowed to react completely until the liquid has become clear and colourless and has a pH below 1, and virtually no more gas bubbles are formed, and the supernatant liquid is separated off from the resulting sediment, which is discarded, the temperature during the entire preparation time being maintained at room temperature or slightly elevated temperature, preferably 20 to 40°C, by cooling or warming.

14. Process for the preparation of the product according to Claim 1 immediately before its use by bringing a metal nitrite which is soluble in aqueous nitric acid into contact with an aqueous nitric acid solution of concentration 6 to 10 millimoles/ml and of ph below 1, characterized in that a porous, pointed rod, intended as applicator, is pretreated or impregnated with a metal nitrite of this type.

15. Means for carrying out the process according to Claim 14, consisting of a porous, pointed applicator rod which is impregnated with a metal nitrite which is soluble in aqeuous nitric acid, in an amount of 0.01 to 5 mg.

**Claims for the Contracting State AT**

1. Process for the preparation of a product for the topical treatment of superficial skin and mucosal lesions of benign and premalignant types and basalomas, characterized in that a metal nitrite which is soluble in aqueous nitric acid is allowed to react, in an amount which corresponds to 0.01 to 5 mg of nitrite ($NO_2$-) per ml of the final solution, with a nitric acid solution of concentration 6 to 10 millimoles/ml or with a concentrated nitric acid solution, and in the latter case the resulting solution is diluted with water to a content of 6 to 10 millimoles of nitric acid per ml and to a pH below 1.

2. Process for the preparation of a product according to Claim 1, which additionally contains at least one organic carboxylic acid which is oxidizable by nitric acid, and at least one metal salt which is non-toxic on external use and is soluble in aqueous nitric acid at a pH below 1, characterized in that the said organic carboxylic acid(s) and the said metal salt(s) are dissolved in water, the aqueous solution is adjusted, by addition of a concentrated nitric acid solution and, if necessary, dilution with water, to a nitric acid concentration of 6 to 10 millimoles/ml and a pH below 1, and is allowed to react with a metal nitrite which is soluble in aqeuous nitric acid, in an amount which corresponds to 0.01 to 5 mg of nitrite per ml of the final solution.

3. Process according to Claim 2, characterized in that the said organic carboxylic acid is an aliphatic hydroxy acid, keto acid or unsaturated acid.

4. Process according to Claim 2 or 3, characterized in that the said organic carboxylic acid is oxalic acid or lactic acid.

5. Process according to Claim 2, characterized in that the said metal salt is one of copper, silver, cadmium and/or zinc.

6. Process according to Claims 4 and 5, characterized in that the said organic carboxylic acid is oxalic acid or lactic aicd, and the said metal salt is a copper and/or a cadmium salt.

7. Process for the preparation of a product according to Claim 1, which additionally contains an organic acid having a keratolytic action, characterized by appropriate addition.

8. Process according to Claims 6 and 7, characterized in that, in arbitrary sequence, copper ions, cadmium ions, concentrated nitric acid, lactic acid or a lower alkyl lactate, oxalic acid or a di(lower alkyl) oxalate, acetic acid or a lower alkyl acetate, and, where appropriate, ethanol, are mixed together and allowed to react completely, at a temperature of 20 to 100°C, until the liquid has become clear and colourless and has a pH below 1, and virtually no more gas bubbles are formed, and the supernatant liquid is separated off from the resulting sediment, which is discarded.

9. Process according to Claim 8, characterized in that concentrated nitric acid, a water-soluble copper(II) salt or metallic copper, with or without a water-soluble cadmium salt or metallic cadmium, are together dissolved and allowed to react, the resulting solution is mixed and allowed to react with lactic acid or ethyl lactate and, possibly, with aqueous ethanol, the solution which has formed is mixed with oxalic acid or diethyl oxalate and then with acetic acid or ethyl acetate, and the solution is allowed to react completely until the liquid has become clear and colourless and has a pH below 1, and virtually no more gas bubbles are formed, and the supernatant liquid is separated off from the resulting sediment, which is discarded, the temperature during the entire preparation time being maintained at room temperature of slightly elevated temperature, preferably 20 to 40°C, by cooling or warming.

10. Process for the preparation of the product according to Claim 1 immediately before its use by bringing a metal nitrite which is soluble in aqueous nitric acid into contact with an aqueous nitric acid solution of concentration 6 to 10 millimoles/ml and of pH below 1, characterized in that a porous, pointed rod, intended as applicator, is pretreated or impregnated with a metal nitrite of this type.

11. Means for carrying out the process according to Claim 10, consisting of a pourous, pointed applicator rod which is impregnated with a metal nitrite which is soluble in aqueous nitric acid, in an amount of 0.01 to 5 mg.

**Revendications pour les Etats contractants BE, CH, DE, FR, GB, IT, LI, NL, SE**

1. Préparation pour le traitement local d'altérations superficielles de la peau et des muqueuses de nature bénigne et prémaligne et des basalomes, caractérisée en ce qu'elle consiste en une solution aqueuse qui contient

(1) de l'acide nitrique en concentration et quantité telles qu'elles confèrent à la solution un pH inférieur à 1 et un équivalent d'acidité de 6 à 10 millimoles/ml, et

(2) un nitrite métallique soluble dans l'acide nitrique aqueux, ou de l'acide nitreux, en une quantité qui correspond à 0,01–5 mg de nitride par ml.

2. Préparation selon la revendication 1, caractérisée en ce que la solution aqueuse contient, en outre, au moins un acide organique carboxylique oxydable par l'acide nitrique.

3. Préparation selon la revendication 2, caractérisée en ce que ledit acide organique carboxylique est un hydroxyacide aliphatique, un acide cétonique aliphatique ou un acide insaturé aliphatique.

4. Préparation selon la revendication 2 ou 3, caractérisée en ce que ledit acide organique carboxylique est l'acide oxalique ou l'acide lactique.

5. Préparation selon la revendication 1, caractérisée en ce que la solution aqueuse contient, en outre, au moins un sel métallique non toxique en application externe, soluble dans l'acide nitrique aqueux à un pH inférieur à 1.

6. Préparation selon la revendication 5, caractérisée en ce que ledit sel métallique est un sel de cuivre, d'argent, de cadmium et/ou de zinc.

7. Préparation selon la revendication 2, caractérisée en ce que la solution aqueuse contient, en outre, au moins un sel métallique ayant les propriétés mentionnées à la revendication 5.

8. Préparation selon la revendication 4, caractérisée en ce que la solution aqueuse contient, en outre, un sel de cuivre et/ou un sel de cadmium ayant les propriétés mentionnées à la revendication 5.

9. Préparation selon la revendication 1, caractérisée en ce que la solution aqueuse contient, en outre, un acide organique carboxylique ayant un effet kératolytique.

10. Procédé de fabrication de la préparation selon la revendication 1, caractérisé en ce que l'on fait réagir un nitrite métallique soluble dans l'acide nitrique aqueux, en une quantité qui corresponde à 0,01–5 mg de nitrite ($NO_2^{\ominus}$) par ml de la solution finale, avec une solution d'acide nitrique d'une concentration de 6 à 10 millimoles/ml ou avec une solution d'acide nitrique plus concentrée et, dans le dernier cas, on dilue à l'eau la solution obtenue jusqu'à une teneur en acide nitrique de 6 à 10 millimoles par ml et un pH inférieur à 1.

11. Procédé de fabrication de la préparation selon la revendication 7 ou 8, caractérisé en ce que l'on dissout dans l'eau ledit (lesdits) acide(s) organique(s) carboxylique(s) et ledit ou lesdits sels métalliques, on ajuste la solution aqueuse à une concentration en acide nitrique de 6 à 10 millimoles/ml et un pH inférieur à 1 par addition d'une solution concentrée d'acide nitrique et dilution éventuelle à l'eau et l'on fait réagir avec un nitrite métallique soluble dans l'acide nitrique aqueux, en une quantité qui corresponde à 0,01–5 mg de nitrite par ml de la solution finale.

12. Procédé de fabrication d'une préparation selon les revendications 8 et 9, caractérisé en ce que l'on mélange ensemble dans un ordre quelconque des ions cuivre, des ions cadmium, de l'acide nitrique concentré, de l'acide lactique ou un ester alcoylique inférieur de l'acide lactique, de l'acide oxalique ou un ester dialcoylique inférieur de l'acide oxalique, de l'acide acétique ou un ester alcoylique inférieur de l'acide acétique et éventuellement de l'éthanol et on les fait réagir complètement, à une température de 20 à 100 °C, jusqu'à ce que le liquide soit devenu clair et incolore et qu'il présente un pH inférieur à 1 et qu'il ne se forme pratiquement plus de bulles de gaz, et l'on sépare le liquide surnageant du dépôt qui s'est formé et que l'on jette.

13. Procédé selon la revendication 12, caractérisé en ce que l'on dissout ou fait réagir ensemble de l'acide nitrique concentré, un sel de cuivre(II) soluble dans l'eau ou du cuivre métallique avec ou sans sel de cadmium soluble dans l'eau ou du cadmium métallique, on traite la solution formée par de l'acide lactique ou un ester alcoylique de l'acide lactique et éventuellement par de l'éthanol aqueux et on fait réagir, on traite la solution obtenue par de l'acide oxalique ou de l'ester diéthylique de l'acide oxalique, puis par de l'acide acétique ou de l'ester éthylique de l'acide acétique et l'on fait réagir la solution complètement, jusqu'à ce que le liquide soit devenu clair et incolore et qu'il présente un pH inférieur à 1 et qu'il ne se forme pratiquement plus de bulles de gaz, et l'on sépare le liquide surnageant du dépôt qui s'est formé et que l'on jette, la température étant maintenue pendant toute la durée de la fabrication à la valeur ambiante ou à une valeur légèrement élevée, de préférence à 20–40 °C, par refroidissement ou par chauffage.

14. Procédé de fabrication de la préparation selon la revendication 1 immédiatement avant son application, par mise en contact d'un nitrite métallique soluble dans l'acide nitrique aqueux et d'une solution aqueuse d'acide nitrique d'une concentration de 6 à 10 millimoles/ml et d'un pH inférieur à 1, caractérisé en ce que l'on soumet à un traitement préalable ou imprègne au moyen d'un tel nitrite métallique un bâtonnet poreux et pointu destiné à servir d'applicateur.

15. Moyen d'exécution du procédé selon la revendication 14, consistant en un bâtonnet applicateur poreux et pointu, qui est imprégné d'un nitrite métallique soluble dans une solution aqueuse d'acide nitrique, en une quantité de 0,01 à 5 mg.

**Revendications pour l'Etat contractant AT**

1. Procédé de fabrication d'une préparation pour le traitement local d'altération superficielles de la peau et des muqueuses de nature bénigne et prémaligne et des basalomes, caractérisé en ce que l'on fait réagir un nitrite métallique soluble dans l'acide nitrique aqueux, en une quantité qui corresponde à 0,01–5 mg de nitrite ($NO_2^-$) par ml de la solution finale, avec une solution d'acide nitrique d'une concentration de 6 à 10 millimoles/ml ou avec une solution d'acide nitrique plus concentrée et, dans le dernier cas, on dilue à l'eau la solution obtenue jusqu'à une teneur en acide nitrique de 6 à 10 millimoles par ml et un pH inférieur à 1.

2. Procédé de fabrication d'une préparation selon la revendication 1, qui contient, en outre, au moins un acide organique carboxylique oxydable par l'acide nitrique et au moins un sel métallique non toxique en application externe, soluble dans l'acide nitrique aqueux à un pH inférieur à 1, caractérisé en ce que l'on dissout dans l'eau ledit (lesdits) acide(s) organique(s) carboxylique(s) et ledit ou lesdits sels métalliques, on ajuste la solution aqueuse à une concentration en acide nitrique de 6 à 10 millimoles/ml et un pH inférieur à 1 par addition d'une solution concentrée d'acide nitrique et dilution éventuelle à l'eau et l'on fait réagir avec un nitrite métallique soluble dans l'acide nitrique aqueux, en une quantité qui corresponde à 0,01–5 mg de nitrite par ml de la solution finale.

3. Procédé selon la revendication 2, caractérisé en ce que ledit acide organique carboxylique est un hydroxyacide aliphatique, un acide cétonique aliphatique ou un acide insaturé aliphatique.

4. Procédé selon la revendication 2 ou 3, caractérisé en ce que ledit acide organique carboxylique est l'acide oxalique ou l'acide lactique.

5. Procédé selon la revendication 2, caractérisé en ce que ledit sel métallique est un sel de cuivre, d'argent, de cadmium et/ou de zinc.

6. Procédé selon les revendications 4 et 5, caractérisé en ce que ledit acide organique carboxylique est l'acide oxalique ou l'acide lactique et ledit sel métallique est un sel de cuivre et/ou un sel de cadmium.

7. Procédé de fabrication d'une préparation selon la revendication 1, qui contient, en outre, un acide organique carboxylique ayant un effet kératolytique, caractérisé par l'addition correspondante.

8. Procédé selon les revendications 6 et 7, caractérisé en ce que l'on mélange ensemble dans un ordre quelconque des ions cuivre, des ions cadmium, de l'acide nitrique concentré, de l'acide lactique ou un ester alcoylique inférieur de l'acide lactique, de l'acide oxalique ou un ester dialcoylique inférieur de l'acide oxalique, de l'acide acétique ou un ester alcoylique inférieur de l'acide acétique et éventuellement de l'éthanol et on les fait réagir complètement, à une température de 20 à 100 °C, jusqu'à ce que le liquide soit devenu clair et incolore et qu'il présente un pH inférieur à 1 et qu'il ne se forme pratiquement plus de bulles de gaz, et l'on sépare le liquide surnageant du dépôt qui s'est formé et que l'on jette.

9. Procédé selon la revendication 8, caractérisé en ce que l'on dissout ou fait réagir ensemble de l'acide nitrique concentré, un sel de cuivre(II) soluble dans l'eau ou du cuivre métallique avec ou sans sel de cadmium soluble dans l'eau ou du cadmium métallique, on traite la solution formée par de l'acide lactique ou un ester alcoylique de l'acide lactique et éventuellement par de l'éthanol aqueux et on fait réagir, on traite la solution obtenue par de l'acide oxalique ou de l'ester diéthylique de l'acide oxalique, puis par de l'acide acétique ou de l'ester éthylique de l'acide acétique et l'on fait réagir la solution complètement, jusqu'à ce que le liquide soit devenu clair et incolore et qu'il présente un pH inférieur à 1 et qu'il ne se forme pratiquement plus de bulles de gaz, et l'on sépare le liquide surnageant du dépôt qui s'est formé et que l'on jette, la température étant maintenue pendant toute la durée de la fabrication à la valeur ambiante ou à une valeur légèrement élevée, de préférence à 20–40 °C, par refroidissement ou par chauffage.

10. Procédé de fabrication de la préparation selon la revendication 1 immédiatement avant son application, par mise en contact d'un nitrite métallique soluble dans l'acide nitrique aqueux et d'une solution aqueuse d'acide nitrique d'une concentration de 6 à 10 millimoles/ml et d'un pH inférieur à 1, caractérisé en ce que l'on soumet à un traitement préalable ou imprègne au moyen d'un tel nitrite métallique un bâtonnet poreux et pointu destiné à servir d'applicateur.

11. Moyen d'exécution du procédé selon la revendication 10, consistant en un bâtonnet applicateur poreux et pointu, qui est imprégné d'un nitrite métallique soluble dans une solution aqueuse d'acide nitrique, en une quantité de 0,01 à 5 mg.